# EUROPEAN PATENT SPECIFICATION

(11) **EP 2 728 360 B1**
(45) Date of publication and mention of the grant of the patent: **15.03.2023**
(21) Application number: 13190681.0
(22) Date of filing: 29.10.2013
(51) Int. Cl.: G01N 35/00, G01N 33/50

(54) **Sample analyzer**
Probenanalysegerät
Analyseur d'échantillon

(30) Priority: 30.10.2012 JP 2012239643
(43) Date of publication of application: 07.05.2014
(73) Proprietor: SYSMEX CORPORATION, Kobe-shi, Hyogo 651-0073 (JP)
(72) Inventor: Ariyoshi, Shunsuke, Hyogo, 651-0073 (JP); Kawate, Yasunori, Hyogo, 651-0073 (JP); Mizumoto, Toru, Hyogo, 651-0073 (JP)
(74) Representative: Hoffmann Eitle

(56) References cited:
- JP-A- 2010 151 672
- JP-B2- 3 043 155
- US-A1- 2005 175 506
- US-A1- 2006 173 575
- US-A1- 2008 063 570
- US-A1- 2010 114 501

## Description

### FIELD OF THE INVENTION

The present invention relates to a sample analyzer for analyzing samples of blood and the like using a reagent or consumable.

### BACKGROUND

Before a patient undergoes medical treatment, a sample collected from the patient is subjected to clinical examination using a sample analyzer. Such examinations usually are performed during daytime on weekdays when the facility, such as a hospital, is open. When a patient is transported to the facility in an emergency, however, the examination may be performed on a weekday night, the weekend or holiday. On holidays and at night on weekdays, an inexperienced laboratory technician unfamiliar with the operation of the sample analyzer, or a laboratory technician specializing in a different examination, may be called upon to perform a variety of analyses outside her area of expertise. Various techniques have been proposed to avoid operational errors when inexperienced operators perform examinations using sample analyzers.

USP 7,384,601 discloses an automatic analyzer which displays a plurality of buttons used in preparatory operations prior to analysis on a screen along with the preparatory flow so as to avoid omission of a preparatory operation of the sample analyzer relating to reagent replacement, replenishment, calibration and precision management regardless of whether the operator is unfamiliar with the apparatus as in night time operation. USP 7,384,601 further discloses a screen for confirming the reagent status, wherein the screen displays the residual amounts of reagents and consumables in the apparatus.

Japanese Laid Open Patent No. 2008-70321 discloses an automatic analyzing system which displays guidance for operating sequences such as reagent preparatory operations on a screen when an unfamiliar operator logs on to the apparatus. The operating sequences shown on this screen include the sequence of recording a reagent in the apparatus, and cautionary items relating to handling reagent.

US 2010/114501 A1 describes a sample measuring apparatus having a screen including a predetermined display area showing a measurable number of times indicating how many times a measuring unit is able to perform a measurement for a predetermined measurement item by using a plural kinds of reagents held by a reagent holder.

US 2006/173575 A1 describes automated reagent dispensing for tissue stainers. The quantity of reagent in a cartridge can be determined and a history of the quantity is maintained.

JP 3 043155 B2 describes displaying the amount of a reagent required in future in comparison with the current remaining amount of the reagent.

JP 2010 151672 A describes an automatic analyzer provided with a residual amount holding means separately from the automatic analyzer, allowing confirmation of a consumable residual amount and a waste residual amount during a waiting time after inputting a power source of the automatic analyzer or the like.

US 2008/063570 A1 describes a sample analyzer that displays detailed information related to the reagent corresponding to a reagent mark selected by an input device on a display device.

According to the art disclosed in above cited documents, although the burden on inexperienced users can be somewhat reduced when replenishing reagent and consumables, the responsibility associated with the operations is not eliminated. Further, samples to be tested in night or on holidays are emergency samples in many cases. Therefore, even if user is familiar with replenishment of reagents or consumables, it is not preferable to consume time for such operation before testing the emergency samples.

### SUMMARY OF THE INVENTION

The intentions of the prior arts are intended to enable a user handling an analyzer on a holiday or night time to smoothly replenish reagent or consumable even if he/she is unfamiliar with an operation of the analyzer. However, an experienced user who routinely handles the analyzer can reliably perform a replenishment of reagent without operational error. Accordingly, replenishment of reagent should preferably be done by the experienced user before changeover of the work of analyzer is made to an inexperienced user.

The present invention provides an interface that helps a user to smoothly replenish reagent or consumable before changeover to an inexperienced user such a night time user or a holiday user.

This object is accomplished by the sample analyzer of the claim 1. The dependent claims concern particular embodiments.

The sample analyzer of a first aspect comprising a storing means for storing a reagent or consumable used for measuring a sample; a measuring means for measuring a sample using the stored reagent or consumable; a control device for analyzing measurement data obtained by the measurement means and issuing instructions for the measurement means;
and a displaying means for displaying a screen, and characterized in that when the current user presses the logoff button, the displaying means is enabled to display a changeover support screen for helping a changeover of operation of the sample analyzer, the support screen showing a residual amount information related to an amount of remaining reagent or consumable stored in the measurement means.

According to the sample analyzer of the first aspect, user can check, via the support screen, a residual amount of reagent or consumable at the current time. By referring to the support screen before changeover, the user can determine whether sufficient reagent or consumable remains to cover measurements expected to be performed after changeover.

It is preferable that the changeover support screen further includes a performance information relating to a consumption performance of reagent or consumable consumed by the measuring means in a predetermined time period in a past. According to the configuration, the residual amount of reagent or consumable at the present time can be visually compared with the consumption performance of the reagent or consumable consumed in a past. The pre-changeover user therefore can empirically determine whether the remaining reagent or consumable is sufficient for covering examinations expected to be performed during the work shift of the post-changeover user.

It is preferable that the sample analyzer further comprises a means for determining whether the residual amount information is in a predetermined relationship with the performance information, and the changeover support screen includes an information display region for showing a residual amount information and a performance information, and an alert display region for showing an alert, and the displaying means shows an alert related to an insufficiency of the reagent or consumable when the residual amount information is not in the predetermined relationship. The labor of the user determining whether there is a possibility of insufficiency is therefore reduced.

It is preferable that the storing means stores a plurality of reagents or a plurality of consumables, and the changeover support screen includes a performance information and a residual amount information for each of the reagents or consumables stored by the storing means. In this way, among a plurality of reagents or consumables, the user can determine which reagent or consumable should be replenished at a glance on the changeover support screen without changing the screen.

It is preferable that the sample analyzer further comprises an obtaining means for obtaining the consumption performance of the reagent or consumable that have been consumed by the measuring means during a plurality of time periods in a past, and a generating means for generating the changeover support screen using an average value of the consumption performances or a maximum value among the consumption performances obtained by the obtaining means. By using the maximum value or the average value of consumption performance, it can be empirically estimated that the reagent or consumable are sufficient for covering the operation of the sample analyzer after changeover if the residual amount exceeds the maximum value or the average value.

It is preferable that the displaying means highlights the residual amount information when the residual amount of the reagent or consumable does not meet a predetermined threshold value or a threshold value determined based on the consumption performance. The user therefore can readily comprehend which reagent or consumable have insufficient residual amounts.

It is preferable that the performance information is an amount of reagent or consumable which is consumed by the measuring means or a number of measurements performed using the reagent or consumable performed by the measuring means in the predetermined time period. Therefore, user can easily understand how many measurements can be performed with the remaining reagent or consumable.

It is preferable that the displaying means is also enabled to display a specification screen for specifying the time period where the sample analyzer is operated after the changeover; and the displaying means displays, as the performance information, the consumption performance of the reagent or consumable consumed by the measuring means during the time period specified on the specification screen. The time period of the changeover of the operation of the sample analyzer therefore can be specified, and the consumption performance during the same time periods of the past can be comprehended. Accordingly, user can set the time period according to the actual situation of upcoming changeover and can more closely estimate a consumption amount of reagent or consumable.

It is preferable that the specification screen includes as selection options at least a first time period defined as night time and a second time period defined as holiday; the displaying means displays the consumption performance of the reagent or consumable consumed by the measuring means during the first time period of a past day as the performance information, when the first time period is selected in the specification screen, and the displaying means displays the consumption performance of the reagent or consumable consumed by the measuring means during the second time period in a past as the performance information, when the second time period is selected in the specification screen. Therefore, the changeover to night time operation or the changeover to holiday operation of the sample analyzer can be simply performed.

It is preferable that the changeover support screen includes a message input region for inputting a message for the user operating the sample analyzer after changeover. The message input region can be utilized for memorandum.

It is preferable that the changeover support screen includes an error display region for showing information related to failures occurring in the sample analyzer on the day. The user therefore can understand there is a possibility of the same error occurring after the changeover by glancing at the error display region. The pre-changeover user can take steps to prevent the failure during post-changeover operation and can leave the sample analyzer in a good condition for the post-changeover user.

It is preferable that the measuring means includes a part required to be periodically replaced with a new one, the changeover support screen includes a part information region for displaying a durable period of the part or a number of uses of the part, and an elapsed time from an installation of the part or a number of uses of the part. Before the changeover, the user can comprehend that the use time of a part requiring periodic replacement is approaching the service life period, or the number of uses of the part is approaching the available number of uses. Therefore, if needed, user can replace the part before changeover to reduce the risk of a breakdown during night time or holiday operation to the lowest level.

It is preferable that the displaying means displays the changeover support screen when a button for completing a work is operated.

It is preferable that the residual amount information is a number of measurements which can be performed by the measuring means using the remaining reagent or consumable.

It is preferable that the changeover support screen includes a replenishment button, and the displaying means shows a replenishment screen on which information of a reagent or consumable to be replenished is input, when the replenishment button of the changeover support screen is operated. Replenishment of reagent or consumable therefore can be smoothly performed.

### BRIEF DESCRIPTION OF THE DRAWINGS

FIG. 1 is a perspective view showing an external view of an embodiment of the sample analyzer;
FIG. 2 is a plan view of the interior of the measurement section of the embodiment viewed from above;
FIG. 3 shows the menu screen D1 of the embodiment;
FIG. 4 shows the reagent layout screen D2 of the embodiment;
FIG. 5A shows the maintenance screen D3 of the embodiment;
FIG. 5B shows the maintenance screen D4 of the embodiment;
FIG. 5C shows the maintenance screen D5 of the embodiment;
FIG. 6 shows the structure of the measurement section of the embodiment;
FIG. 7 shows the structure of the control device of the embodiment;
FIG. 8A is a conceptual view showing the structure of the reagent residual amount database;
FIG. 8B is a conceptual view showing the structure of the reagent usage amount setting;
FIG. 8C is a conceptual view showing the structure of the error history database;
FIG. 8D is a conceptual view showing the structure of the measurement history database;
FIG. 9 shows a screen D6 which is displayed when the changeover button is pressed in the embodiment;
FIG. 10 shows the changeover support screen D7 of the embodiment;
FIG. 11 is a flow chart showing the process of the control device during changeover in the embodiments;
FIG. 12A shows the warning dialog D8 of the embodiment;
FIG. 12B shows a flow chart showing the display process of the changeover support screen;
FIG. 13A shows the changeover setting screen D9 of the modification;
FIG. 13B shows an example of time periods of night time and holiday;
FIG. 14A shows a modification of list of the changeover support screen;
FIG. 14B shows a modification of list of the changeover support screen;
FIG. 14C shows a modification of list of the changeover support screen;
FIG. 15 shows a list of the changeover support screen of another modification; and
FIG. 16 shows the changeover support screen of another modification;

### EMBODIMENT

Hereinafter an embodiment of the present invention is described referring to the appended drawings.

The sample analyzer of the present embodiment is a blood coagulation analyzer which performs analyses related to clotting function by irradiating light on a measurement sample prepared by adding reagent to a sample (blood plasma), and analyzing the obtained transmission light via a coagulation method, synthetic substrate method, immunoturbidity method, or condensation method. The sample analyzer of the embodiment is described below referring to the drawings.

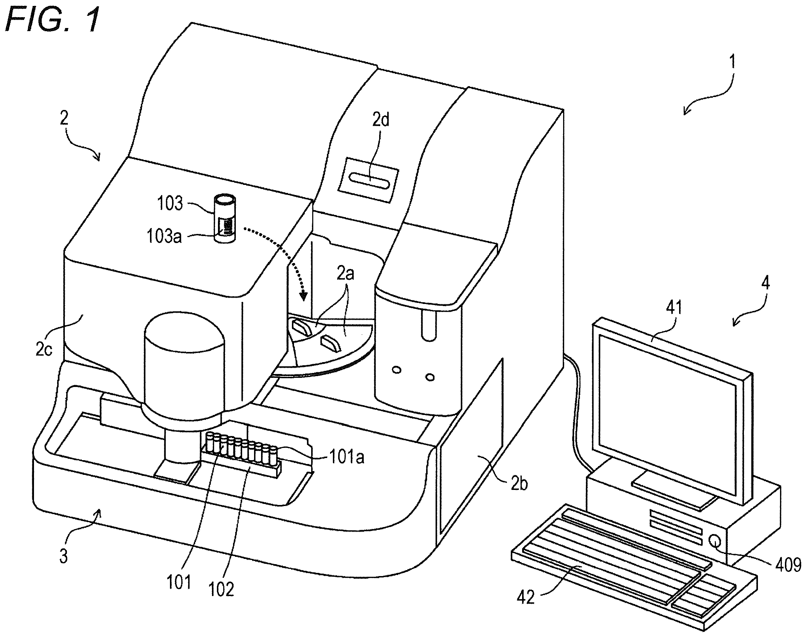

FIG. 1 is an exterior perspective view showing the structure of a sample analyzer 1.

The sample analyzer 1 is provided with a measurement section 2 which optically measures components contained in a sample (blood plasma), sample transport section 3 which is disposed in front of the measurement section 2, and control device 4 which analyzes the measurement data obtained by the measurement section 2 and issues instructions for the measurement section 2.

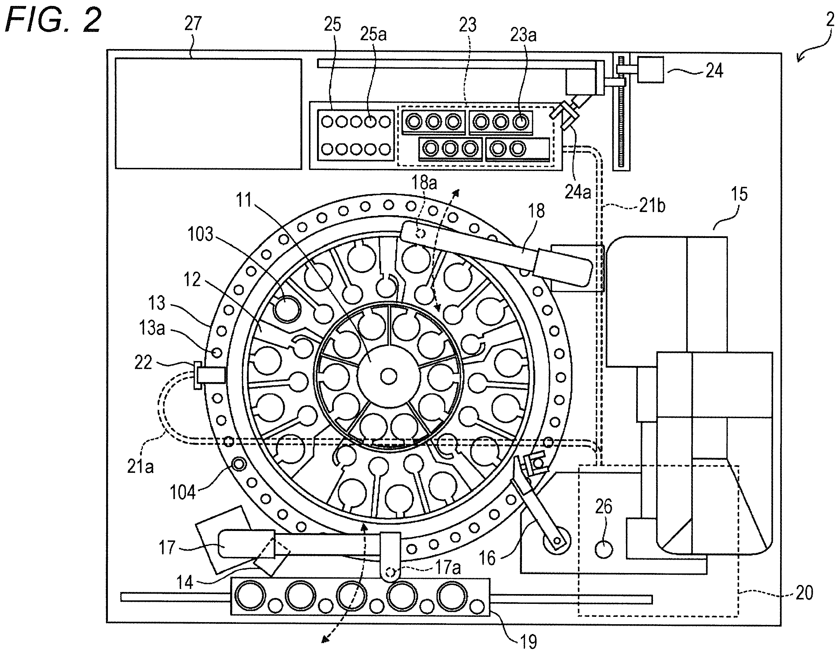

The measurement section 2 has covers 2a, 2b, and 2c, and a power button 2d. The user opens the cover 2a to replace the reagent containers 103 installed on reagent tables 11 and 12 (refer to FIG. 2) with new reagent containers 103, or to add another new reagent container 103.

Note that, in the specifications, the use of the term "replenish" to mean both "replace" and "add" in reference to the reagent containers 103. In other words, replenishment means the work of increasing the residual amount of a reagent or consumable. Note also that such kind of reagent or consumable which does not require a management of lot number or expiry date may be replenished by addition. Examples of reagent which may be extended by addition include washing liquid for washing the pipettes and staining liquid that contains dye material.

Adhered to the reagent container 103 is a barcode label 103a which bears a printed barcode that includes the type of reagent contained therein and the reagent ID, which is a serial number assigned to the reagent. The user opens the cover 2b to replace the lamp unit 20 (refer to FIG. 2), and opens the cover 2c to replace the piercer 17a (refer to FIG. 2). The sample transport section moves the sample container 101 held in a sample rack 102 to the aspirating position of the piercer 17a. The sample container 101 is sealed by a rubber cap 101a.

When an operator uses the sample analyzer 1, the power button 2d of the measurement section 2 is pressed to start the measurement section 2, then the power button 409 of the control device 4 is pressed to start the control device 4. When the control device 4 is started, the login screen is shown on the display section 41. The user logs into the control device 4 by entering a user name and password in the login screen, and begins to use the sample analyzer 1. When the measurement section 2 and control device 4 are started from a logged off condition (no one is logged on from any account) and is used by another user just before, the user logs on to the control device 4 by entering a user name and password on the logon screen shown on the display section 41, and starts to use the sample analyzer 1.

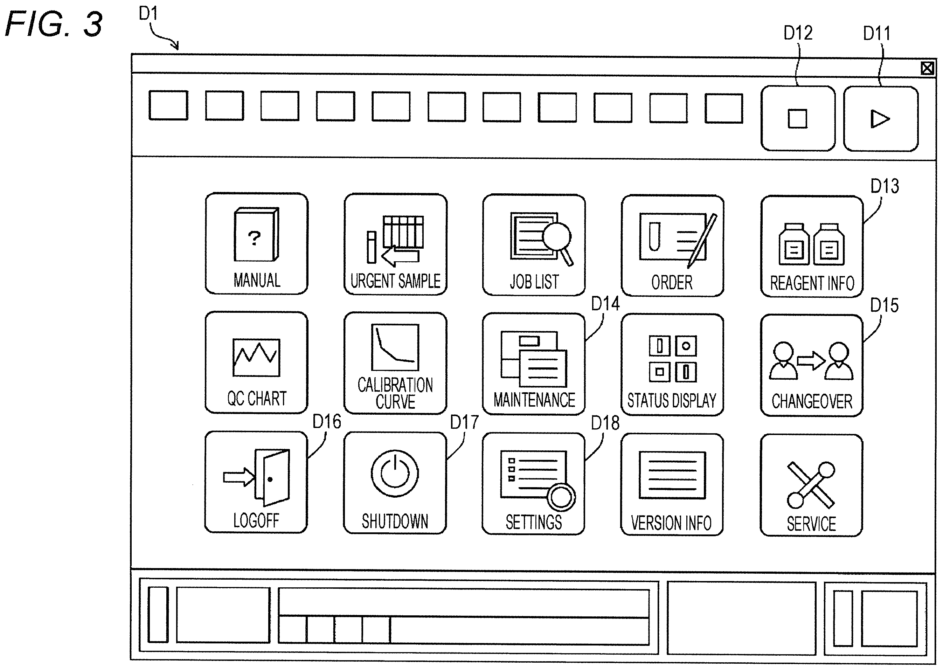

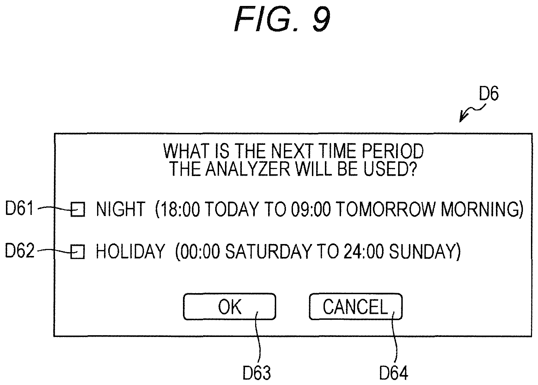

To stop operating the sample analyzer 1, the user presses the shutdown button D17 shown on the display section 41 (refer to FIG. 3) to shutdown the control device 4, and presses the power button 2d of the measurement section 2 to turn off the power to the measurement section 2 and stop using the sample analyzer 1. When another user immediately takes over the operation of the sample analyzer 1, the user presses the logoff button D16 (refer to FIG. 3) shown on the display section 41 to log off from the control device 4 and end his use of the sample analyzer 1. Note that when another user takes over the operation of the sample analyzer 1 in the present embodiment, the user presses the changeover button D15 (refer to FIG. 3) to perform the changeover operation before pressing the logoff button D16 or the shutdown button D17. The changeover operation is described below with reference to FIG. 9 and subsequent drawings.

FIG. 2 is a plan view of the interior of the measurement section 2 viewed from above.

The reagent tables 11 and 12 and cuvette table 13 are annular in shape and rotatable. The reagent containers 103 are installed on the reagent tables 11 and 12. The barcodes of the reagent containers 103 installed on the reagent tables 11 and 12 are read by a barcode reader 14. The information read from the barcode, that is, the reagent type and ID, is input to the control device 4 and stored on a hard disk 404. The cuvette table 13 has a support part 13a configured with a plurality of holes capable of supporting cuvettes 104. New cuvettes 104, which have been loaded in a cuvette supplier 15 by the user, are sequentially moved by the cuvette supplier 15 and installed in the cuvette support part 13a of the cuvette table 13 by a catcher 16.

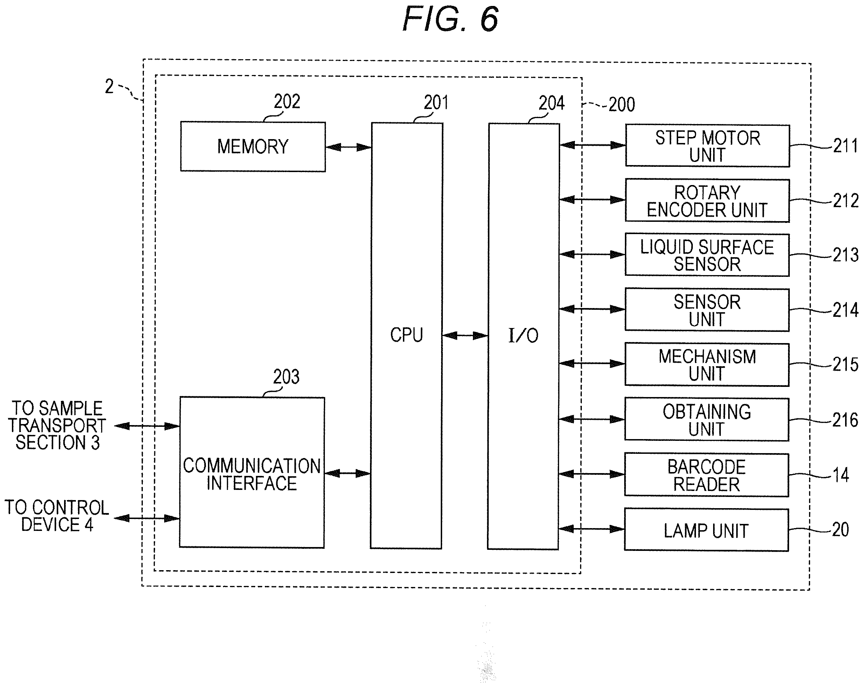

Step motors are respectively connected to the sample dispensing arm 17 and reagent dispensing arm 18 to raise and lower and rotate the arms. A piercer 17a, which is tapered to a point at the tip to be capable of piercing the cap 101a of the sample container 101, is provided at the leading end of the sample dispensing arm 17. A pipette 18a is provided at the leading end of the reagent dispensing arm 18. The tip of the pipette 18a is flat, unlike the piercer 17a. A capacitive type liquid level sensor 213 (refer to FIG. 6) is connected to the pipette 18a.

The lamp unit 20 supplies light of five wavelengths used in the detection of optical signals by the first detection unit 22 and the second detection unit 23. The light emitted by the lamp unit 20 is supplies to the first detection unit 22 and the second detection unit 23 through optical fibers 21a and 21b, respectively.

When the sample container 101 is moved to a predetermined position by the sample transport section 3, the piercer 17a is positioned directly above the sample container 101 by the rotational movement of the sample dispensing arm 17. The sample dispensing arm 17 is then lowered and the piercer 17a penetrates the cap 101a of the sample container 101, and the piercer 17a aspirates the sample held in the sample container 101. When a sample requiring urgent attention is placed in the emergency sample receiver 19, the piercer 17a aspirates the urgent sample by interrupting the processing of the sample supplied from the sample transport section 3. The sample aspirated by the piercer 17a is discharged into a new cuvette 104 on the cuvette table 13.

The cuvette 101 which holds the discharged sample is moved to a position for obtaining optical information by the first detection unit 22 via the rotation of the cuvette table 13. The first detection unit 22 irradiates the sample to which reagent was previously added by light emitted from the lamp unit 20, and obtains the amount of transmitted light (first optical information). The obtained first optical information is transmitted to the control device 4.

When the first optical information is obtained, the cuvette 104 is moved from the support part 13a of the cuvette table 13 to the support part 25a of the heating unit 25 by the catcher 24a of the cuvette transporter 24. The heating unit 25 heats the sample held in the cuvette 104 installed on the support part 25a to about 37 degrees Centigrade. When heating of the sample by the heating unit 25 is completed, the cuvette 104 is again held by the catcher 24a. The cuvette 104 is then disposed at a predetermined position while held by the catcher 24a, and the reagent aspirated by the pipette 18a is discharged therein.

When dispensing the reagent via the pipette 18a, the reagent tables 11 and 12 are first rotated to move the reagent containers 103 containing the reagents corresponding to the measurement item to the aspirating position of the pipette 18a. After the vertical position of the pipette 18a is set at the origin position based on a sensor which detects the origin position, the pipette 18a is lowered until the bottom end of the pipette 18a contacts the liquid surface of the reagent via the liquid level sensor 213. When the bottom end of the pipette 18a contacts the reagent liquid surface, the pipette 18a is lowered to the degree required to aspirate the required amount of reagent. Thereafter, the descent of the pipette 18a is stopped and the reagent is aspirated by the pipette 18a. The reagent aspirated by the pipette 18a is discharged into a cuvette 104 held by the catcher 24a. The sample and reagent within the cuvette 104 are mixed together by the oscillation of the catcher 24a. The preparation of the measurement sample is accomplished in this way.

The cuvette 104 holding the measurement sample is moved to the support part 23a of the second detection unit 23 by the catcher 24a. The second detection unit 23 irradiates the cuvette 104 by light emitted from the lamp unit 20, and obtains the amount of transmitted light (second optical information). The obtained second optical information is transmitted to the control device 4. The control device 4 performs analysis based on the first optical information and second optical information, and shows the analysis results on the display section 41. The measurement of the sample is completed in this way.

The cuvette 104 which is unnecessary after measurement has been completed is moved by the cuvette table 13 and discarded in the discard hole 26 by the catcher 16. The piercer 17a and the pipette 18a are washed using a suitable liquid such as a washing liquid supplied from the fluid section.

FIG. 3 shows the menu screen D1 being displayed on the display section 41 of the control device 4. The menu screen D1 is shown on the display section 41 when a user logs onto the control device 4.

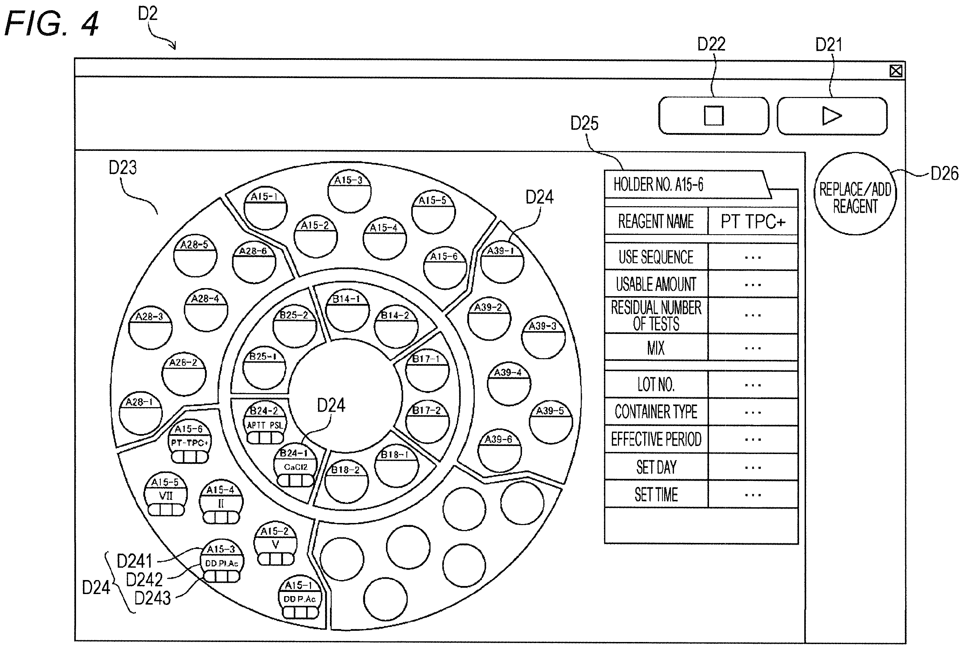

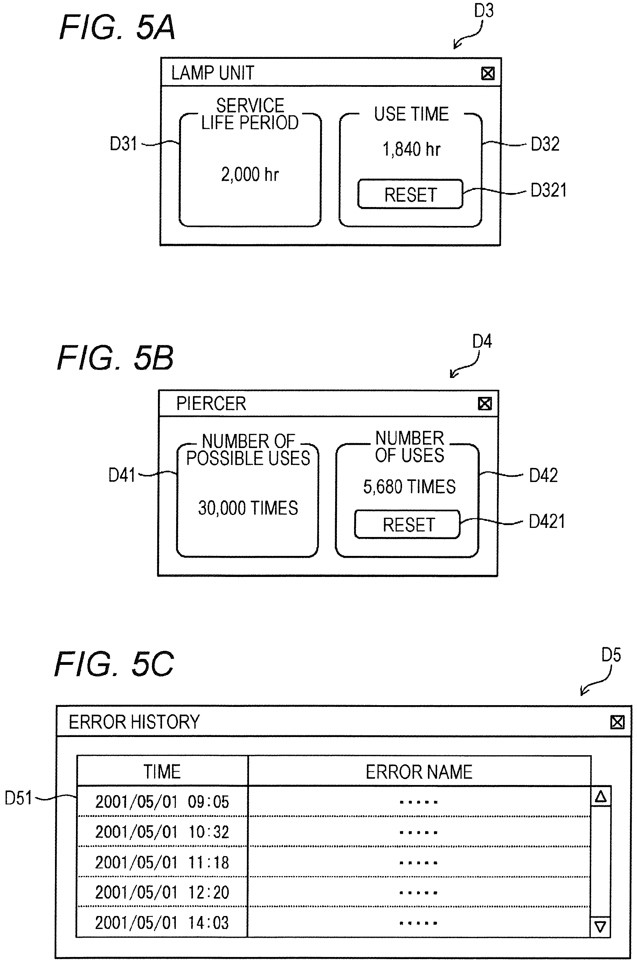

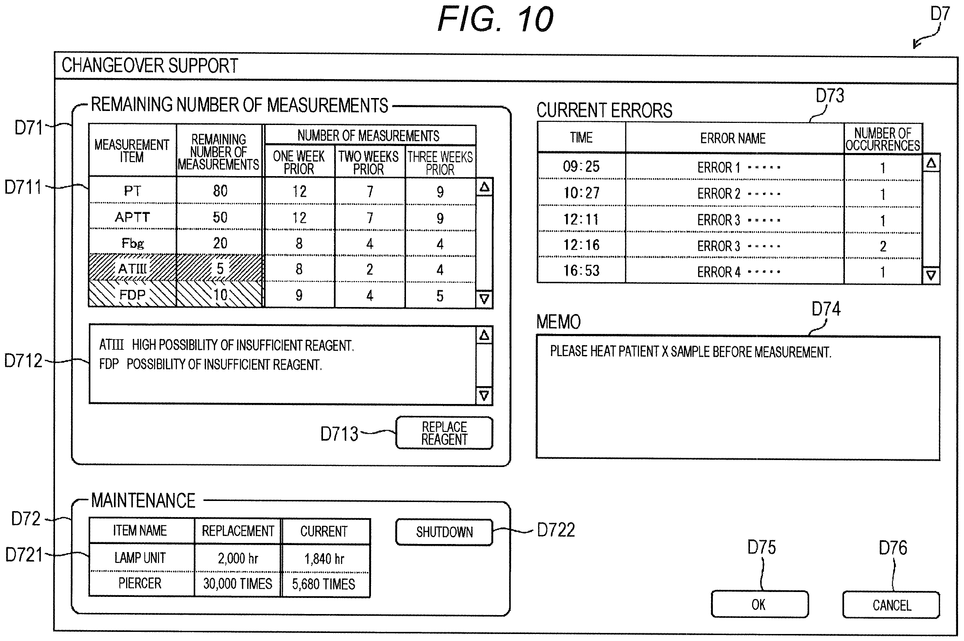

The menu screen D1 includes a button D11 for starting the measurement operation of the measurement section 2 and sample transport unit 3, button D12 for stopping the measurement operation, reagent information button D13 for displaying the reagent layout screen D2 (refer to FIG. 4), maintenance button D14 for displaying the maintenance screens D3 through D5 (refer to FIG. 5A through 5C), changeover button D15, logoff button D16 for displaying the logoff from the control device 4, shutdown button D17 for shutting down the control device 4, and setting button D18 for performing various settings. The changeover button D15 is described later referring to FIGS. 9 and 10.

FIG. 4 shows the reagent layout screen D2 being displayed on the display section 41 of the control device 4.

The reagent layout screen D2 includes buttons D21 and D22 for starting and stopping the measurement operation similar to buttons D11 and D12 of the menu screen D1, reagent layout display region D23, reagent information display region D25, and reagent replace/add button D26 for replenishing the reagent.

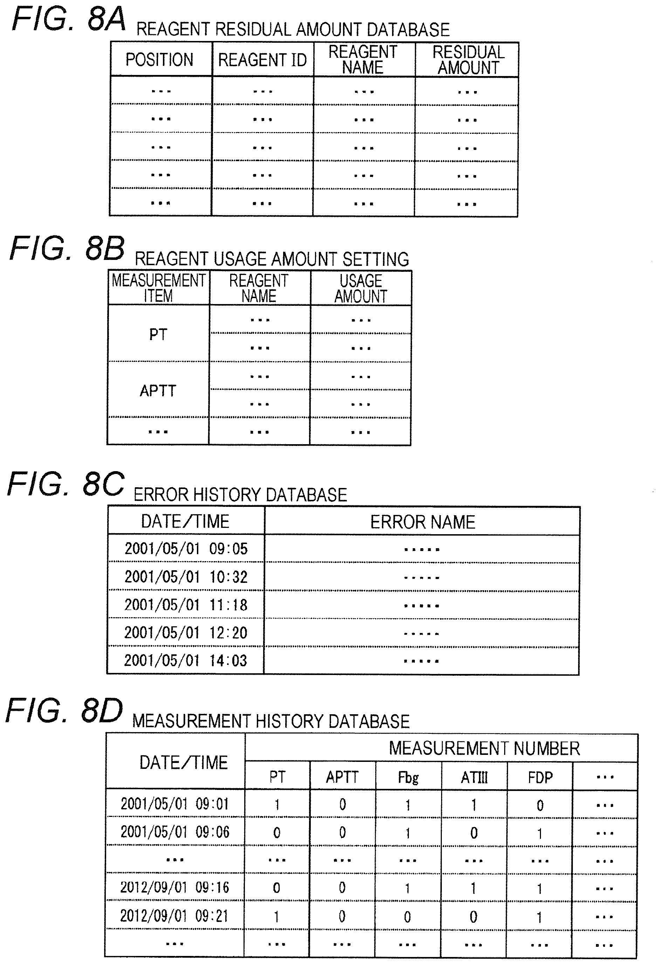

The reagent layout display region D23 displays a reagent layout region D24 which corresponds to the position of the reagent container 103 on the reagent tables 11 and 12. The reagent layout region D24 includes a display part D241 showing the position of the reagent container 103, a display part D242 showing the reagent name of the reagent in the reagent container 103, and a display part D243 showing the residual amount of reagent in the reagent container 103. The residual amount of reagent shown in the display part D243 is based on the residual amount item in the reagent residual amount database (refer to FIG. 8A).

The reagent information display region D25 shows the attribute information (holder number, reagent name, available amount, remaining number of tests and the like) of the reagent container 103 specified by pressing the reagent display region D24. Among the attribute information, the holder number corresponds to the display content of the display part D241, and the available amount corresponds to the display content of the display part D243. Since the reagent is used in a plurality of measurement items, the remaining number of tests shown in the reagent information display region D25 indicates the number of measurements which can be performed using the remaining reagent when the amount of reagent used to measure a specific measurement item is designated as a single measurement.

When the reagent replace/add button D26 is pressed while the reagent display region D24 shows the layout of the reagent containers 103, the reagent table 11 or 12 is rotated, and the reagent container 103 which corresponds to the specified reagent display region D24 is moved directly below the cover 2a. The user therefore can open the cover 2a and replace the reagent container 103. When the reagent replace/add button D26 is pressed while the reagent display region D24 where no reagent container is set is specified, the position on the reagent table 11 or 12 corresponding to the specified position is moved directly below the cover 2a. The user therefore can open the cover 2a and add a new reagent container 103 to the vacant slot.

FIG. 5A through 5C show the maintenance screens D3 through D5 in the display part 41 of the control device 4. The maintenance screens D3 through D5 are respectively displayed by operating buttons in the maintenance specification screen which is displayed by pressing the maintenance button D14 of the menu screen D1.

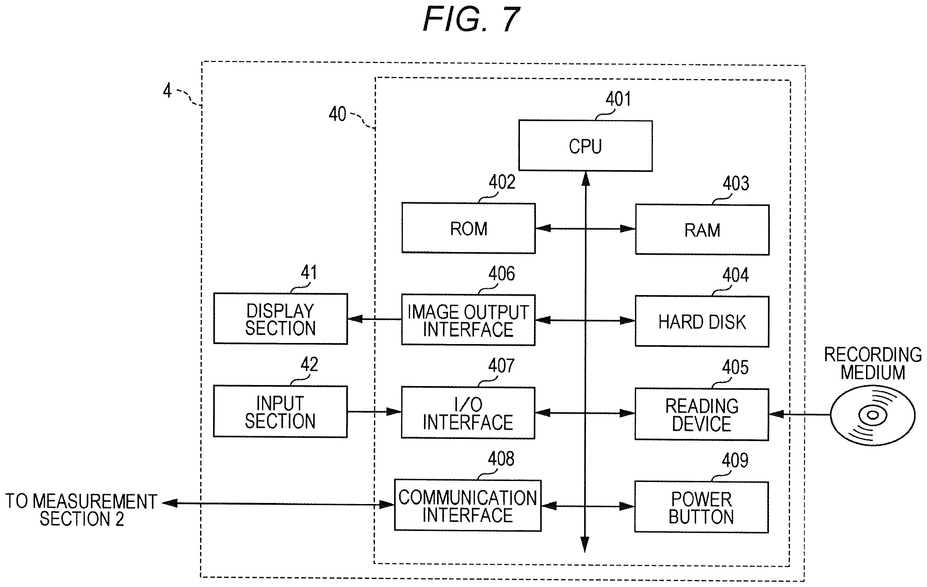

Referring to FIG. 5A, the maintenance screen D3 is a screen for performing maintenance of the lamp unit 20, and includes fields D31 and D32. The field D31 displays the service life period which indicates the standard timing for replacement of the lamp unit 20, and field D32 displays the use time of the currently installed lamp unit 20. Field D32 also includes a reset button D321 for resetting the use time. The service life period and use time are stored on the hard disk 404 (refer to FIG. 7) of the control device 4, and the use time is incremented according to the amount of time the lamp unit 20 is used.

When the user determines that the lamp unit 20 requires replacement by referring to the service life period in field D31 and the use time in field D32, the user returns to the menu screen D1 and presses the shutdown button D17 to shut down the control device 4 and turn off the power supply of the measurement section 2. The user then opens the cover 2b and replaces the lamp unit 20, then restarts the sample analyzer 1 and logs on to the control device 4. The maintenance screen D3 is again displayed and the user presses the reset button D321 to set the use time to 0 hours in the field D32. The replacement of the lamp unit 20 is completed in this way.

Referring to FIG. 5B, the maintenance screen D4 is a screen for performing maintenance of the piercer 17a, and includes fields D41 and D42. The field D41 displays the number of possible uses (i.e. available number of times) which indicates the standard timing for replacement of the piercer 17a, and field D42 displays the number of times used of the currently installed piercer 17a. Field D42 also includes a reset button D421 for resetting the number of times used. The number of possible uses and the number of times used are stored on the hard disk 404 (refer to FIG. 7) of the control device 4, and the number of times used is incremented each time the sample dispensing arm 17 is raised/lowered and the piercer 17a penetrates the cap 101a of the sample container 101.

When the user determines that the piercer requires replacement by referring to the number of possible uses in field D41 and the number of times used in field D42, the user returns to the menu screen D1 and presses the shutdown button D17 to shut down the control device 4 and turn off the power supply of the measurement section 2. The user then opens the cover 2c and replaces the piercer 17a, then restarts the sample analyzer 1 and logs on to the control device 4. The maintenance screen D4 is again displayed and the user presses the reset button D421 to set the number of uses to 0 times in the field D42. The replacement of the piercer 17a is completed in this way.

Referring to FIG. 5C, the maintenance screen D5 is a screen which shows the history of error generated in the measurement section 2, and includes a list D51. The error history shown in list D51 is displayed based on the error history database (refer to FIG. 8C). The user can handle problems appropriately by referring to the list D51 based on past errors.

FIG. 6 is a shows the structure of the measurement section 2.

The measurement section 2 includes a controller 200, step motor unit 211, rotary encoder unit 212, liquid surface sensor 213, sensor unit 214, mechanism 215, barcode reader 14, and lamp unit 20. The controller 200 includes a CPU 201, memory 202, communication interface 203, and I/O interface 204.

The CPU 201 executes computer programs stored in the memory 202. The memory 202 is configured by a ROM, RAM, hard disk or the like. The CPU 201 also drives the sample transporter 3 and sends and receives instruction signals and data to/from the control device 4 through the communication interface 203. The CPU 201 also controls each unit in the measurement section 2 and receives signals output by each unit through the I/O interface 204.

The step motor unit 211 includes the reagent tables 11 and 12, cuvette table 13, catcher 16 sample dispensing arm 17, reagent dispensing arm 18, cuvette mover 24, and respective step motors for drive each. The rotary encoder unit 212 includes rotary encoders which output a pulse signal corresponding to the amount of rotational displacement of each step motor incorporated in the step motor unit 211.

The liquid surface sensor 213 is connected to the pipette 18a installed on the leading end of the reagent dispensing arm 18, and detects when the bottom end of the pipette 18a touches the liquid surface of the reagent. The sensor unit 214 includes a sensor for detecting when the position of the pipette 18a in the vertical position is at the origin position, and a sensor for detecting when the power supply button 2d is pressed. The mechanism unit 215 includes devices for driving the cuvette supplier unit 15, urgent sample receiver unit 19, heating unit 25, and fluid unit 27, and a vacuum source for supplying pressure to the piercer 17a and pipette 18a to perform the dispensing operations of the piercer 17a and pipette 18a. The detection unit 216 includes a first detector 22 and a second detector 23.

FIG. 7 is a shows the structure of the control device 4.

The control device 4 is configured by a personal computer that includes a main body 40, and display section 41, and input section 42. The main body 40 has a CPU 401, ROM 402, RAM 403, hard disk 404, reading device 405, image output interface 406, I/O interface 407, communication interface 408, and power button 409.

The CPU 401 is capable of executing a computer program stored in the ROM 402 and a computer program loaded in the RAM 403. The RAM 403 is used when reading the computer program stored in the ROM 402 and recorded on the hard disk 404. The RAM 403 is also used as the work area of the CPU 401 when the CPU 401 executes the computer programs.

An operating system, computer programs executed by the CPU 401, reagent residual amount database, reagent amount used setting database, error history database, measurement history database (refer to FIG. 8A through 8D), and setting content of the control device 4 are stored on the hard disk 404. The reader 405 is a CD drive or DVD drive capable of reading computer programs and data recorded on a recording medium.

The image output interface 406 outputs image signals corresponding to the image data to the display section 41, and the display section 41 displays the image based on the image signals output from the image output interface 406. The user inputs instructions through the input section 42, and the I/O interface 407 receives the signals input through the input section 42. The communication interface 408 is connected to the measurement section 2, and the CPU 401 sends and receives instruction signals and data to/from the measurement section 2 through the communication interface 408.

FIG. 8A is a conceptual view showing the structure of the reagent residual amount database.

The reagent residual amount database includes position items which indicate the positions of the reagent containers 103 held in the reagent tables 11 and 12, reagent ID items indicating the reagent IDs, reagent name items indicating the name of the reagents, and residual amount items indicating the remaining amounts (mL) of reagent. The residual amount items are updated when reagent is replaced or added to, and when reagent is aspirated.

Relational expressions of liquid surface height and reagent residual amount (volume) are stored for various types of reagents on the hard disk 404 of the control device 4. When the reagent replace/add button D26 is pressed, the pipette 18a is inserted into each reagent container 103, and the height position of the liquid surface is detected. The residual amount (volume) of the reagent is calculated based on the relational expression stored on the hard disk 404 and the detected liquid height position, and the numerical value of the residual amount item is updated. When reagent is aspirated, the aspirated amount of reagent (amount used) is subtracted from the residual amount stored in the residual amount item of the reagent residual amount database at that time, and the resulting value is used as the new reagent residual amount to update the residual amount item. The residual amounts of all reagent containers 103 on the reagent tables 11 and 12 are therefore stored in the reagent residual amount database.

Note that the height position of the liquid surface is obtained based on the amount of rotational displacement (number of pulses output by the rotary encoder) of the step motor connected to the reagent dispensing arm 18 after the pipette 18a is set at the origin position until the liquid surface of the reagent is contacted. The amount of reagent required for each measurement item is predetermined based on the reagent use amount setting shown in FIG. 8B. The reagent use amount setting includes the measurement item, reagent name item indicating the name of the reagent required for the measurement item, and the use amount item indicating the amount of reagent required for a single measurement.

FIG. 8C and 8D are conceptual views respectively showing the structures of the error history database and the measurement history database.

The error history database includes a date and time item indicating the date and time the error occurred, and an error name item indicating the name of the error. The measurement history database includes a date and time item indicating the date and time the measurement was completed, and a measurement number item indicating the number of measurements of the measurement item for each sample. Note that the date and time item is the date and time the final measurement item was completed when a single sample is dispensed to a plurality of cuvettes 104 and a plurality of measurements are performed.

In general, many samples are used in routine examinations (regularly conducted examinations) during day time on weekdays. In order to assign staff to efficiently perform examinations, at least one technician who has received special training in the operation of the sample analyzer 1 is typically assigned with respect to the sample analyzer.

On the other hand, routine examinations are not performed on weekday nights or holidays. Examinations are performed only in irregular cases, for example, cases of examining a sample from patient arriving at the facility under emergency condition. Therefore, on nights and holidays, many facilities assigns an inexperienced technician on duty, or assigns a specialist technician of a specific filed of analyzer (for example, blood cell counter, biochemical analyzer) to cover another filed of analyzers such as a blood coagulation analyzer, in order to avoid excess staff.

At night and on holidays, it frequently happens that a user who is unfamiliar with the operation of the sample analyzer 1 will log on to the control device 4 to operate the sample analyzer 1. When reagent becomes depleted or the lamp unit 20 or piercer 17a breakdown at night or on a holiday, the reagent can be replenished and the lamp unit 20 or piercer 17a can be replaced smoothly if the user is familiar with the operation of the apparatus. However, these operations are not easy for a user who is unfamiliar with these operations. Generally, the examinations occurring at night and on holidays have a high degree of urgency and taking time to perform the corrective operations is undesirable.

To avoid depleting the reagent at night time or on holidays, the person in charge during the daytime must replenish the reagent and/or the consumable before the changeover to provide sufficient residual amounts for examinations performed at night and on holidays.

One method of preventing inadequate amount of reagents is to consider maintaining the reagent and consumables at full capacity, but this is impractical due to the problem of the expiration date of each reagent. In the case of many reagents, the expiration period is the period during which the reagent can be consumed after the reagent is opened. The expiration period after opening of some kinds of reagents is short at approximately 30 days, which makes it desirable to delay opening the reagent as possible.

In view of these facts, the decision as to whether reagent replenishment is required is generally decided in this way: replenishment is not required when the amount of residual reagent is sufficient to minimum requirements at night or on holidays, however, replenishment is required when the residual amount of reagent is insufficient for the minimum requirements.

The user on duty during day time normally cannot know the frequency with which each measurement item will be performed at night and on holidays. How much residual amount will be sufficient night time and holiday examinations therefore cannot be estimated, or if estimated, the past examination history must be checked. Moreover, and inconveniently, since the current residual amount of reagent and consumables must be known to determine whether replenishment is required, just how much reagent and consumables are currently in the sample analyzer 1 must be checked.

In the present embodiment, the past consumption amount of the reagent during the night time period and the current residual amount of the reagent can be compared at a glance by aggregating and displaying the data in a single screen, for helping the user on duty during the daytime to perform the changeover to a night time or holiday (hereinafter referred to as "night time period") user of the sample analyzer 1. Specifically, the changeover support screen D7 (refer to FIG. 10) is displayed when the changeover button D15 shown in FIG. 3 is pressed by the daytime user who is ending his work shift. The user performs the changeover work by replenishing reagent and consumables after referring to the changeover support screen D7. Note that in the specifications, the phrase "time period" may include an hour interval within a 24 hour period, as well as a period exceeding 24 hours.

FIG. 9 shows the screen D6 which is displayed when the changeover button D15 is pressed. The screen D6 is a screen for receiving user instruction to perform the changeover for one of the time periods. When the user on weekday daytime duty performs the changeover to a user on night duty, a check is entered in the check box D61 corresponding to "night time" and the OK button is then pressed. When the next day is a holiday (Saturday or Sunday) and the daytime user performs the changeover to a holiday user, a check is entered in the check box D62 corresponding to "holiday" and then the OK button is pressed. When the cancel button D64 is pressed, the entered content is deleted and the screen returns to the menu screen D1.

FIG. 9 shows the changeover support screen D7 for the changeover to the night time duty user when "night time" is selected. The changeover support screen D7 for the changeover to the holiday duty user is shown when "holiday" is selected. Note that in the present embodiment night time means the time period from 18:00 hours to 09:00 hours the next day. Information related to the consumption amount of the reagent consumed in the time period from 18:00 hours to 09:00 hours in the past one day is shown in the changeover support screen D7 for changeover to the night duty user. In the present embodiment, "holiday" means the time period from 00:00 hours Saturday to 24:00 hours on Sunday. Accordingly, when the weekday daytime user presses the changeover button D15 and selects the "holiday" check box 62 in the screen D6, information related to the consumption amount of reagent consumed from the past Saturday to Sunday is displayed on the changeover support screen.

The following description pertains to the example shown in the screen D6 of FIG. 9 when the "night time" has been selected.

FIG. 10 shows the changeover support screen D7 being displayed on the display section 41 of the control device 4.

Region D71 includes list D711, notification region D712, and reagent replacement button D713. The list D711 shows the remaining number of measurements and past number of measurements for each measurement item.

The remaining number of measurements is the number of measurements possible using the reagent at the current time; the value is obtained by dividing the volume of reagent stored in the residual amount item of the reagent residual amount database (FIG. 8A) by the reagent usage amount (FIG. 8B) for a single measurement. The number of past measurements is the number of measurements performed during the night time (18:00 hours to 09:00 hours the next day) period of the day of the week corresponding to the current day one week previously, two weeks previously, and three weeks previously. For example, if the current day is March 28, the previous time periods are from 18:00 hours on March 21 to 09:00 hours on March 22, 18:00 hours on March 14 to 09:00 hours on March 15, and 18:00 hours on March 7 to 09:00 hours on March 8. The number of past measurements is calculated by the CPU 401 by extracting the measurement histories which include the time and date from the measurement history database, and adding them, with the resulting total value displayed.

For a specific measurement item, when the number of measurements currently remaining are less than the maximum value of the past number of measurements, the measurement item and the number of measurements are highlighted in a dark color on the display. The notification region D712 displays a message alerting that there is a high possibility that reagent is insufficient for this measurement item in the specified time period (that is, 18:00 hours today until 09:00 hours tomorrow) in FIG. 9.

For a particular measurement item, when the current number of measurements remaining is less than the value obtained by adding a predetermined value N (for example, 2) to the maximum value of the past number of measurements, this measurement item and the number of measurements are highlighted in a light color in the display. The notification region D712 also display a message alerting of the possibility of insufficient reagent in the specified time period for this measurement item.

When the user refers to the list D711 and the notification region D712 and determines that there is a possibility of reagent insufficiency, the user presses the reagent replacement button D713 and the reagent layout screen D2 shown in FIG. 4 is displayed. The user then replaces the reagent container 103 or adds to the reagent container 103 through the reagent layout screen D2 as previously described. When the replacement or addition of the reagent container 103 is completed and the reagent layout screen D2 is closed, the content in the region D71 is updated on the display.

The region D72 includes a list D721 and a shutdown button D722. The list D721 shows information similar to the maintenance screens D3 and D4, that is, the service life period and use time of the lamp unit 20, and the number of possible uses and usage number of the piercer 17a.

When the user refers to the list D721 and determines that replacement of the lamp unit 20 or the piercer 17a is necessary, the user presses the shutdown button D722 and the control device 4 shuts down and the power to the measurement section 2 is turned off. The user then replaces the lamp unit 20 or the piercer 17a via the replacement sequence described above, then restarts the sample analyzer 1 and logs on to the control device 4. In this case, the control device 4 is configured to automatically display the maintenance screens D3 and D4 after log on, and the user presses either the reset button D321 or the reset button D421 to reset the usage time or the usage number. The control device 4 is also configured to automatically open the changeover support screen D7 when the maintenance screens D3 and D4 are closed. In this case, the usage time or the usage number are in the reset state in the list D721.

The list D73 shows only errors generated during logon by the current from the error history database. The list D73 includes the generated number item indicating the number of generated errors. The generated number item shows the number of the generated error among the similar errors generated during logon by the current user.

The input region D74 is a region in which the user can enter text. When the user presses the OK button D75 after entering text in the input region D74, the text data entered in the input region D74 is stored on the hard disk 404 of the control device 4. When a subsequent user logs on after the changeover, a screen which includes the text data entered in the input region D74 is shown on the display section 41. In this way the user can communicate with the subsequent user.

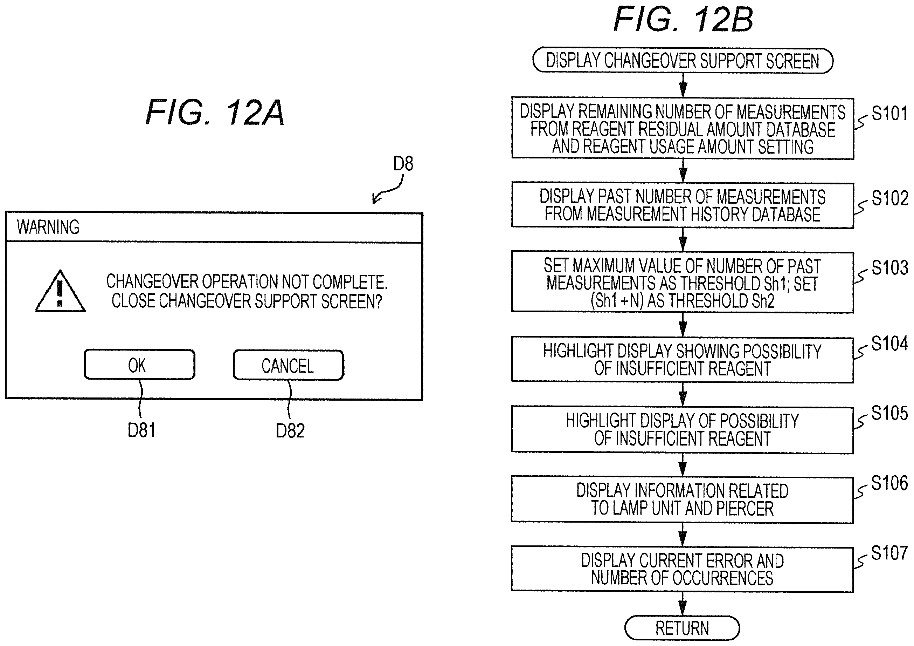

When the user presses the OK button D75 regardless of the message alerting that reagent replenishment is necessary in the region D71, the warning dialog D8 is displayed (refer to FIG. 12A). When the cancel button D76 is pressed, the changeover support screen D7 is closed.

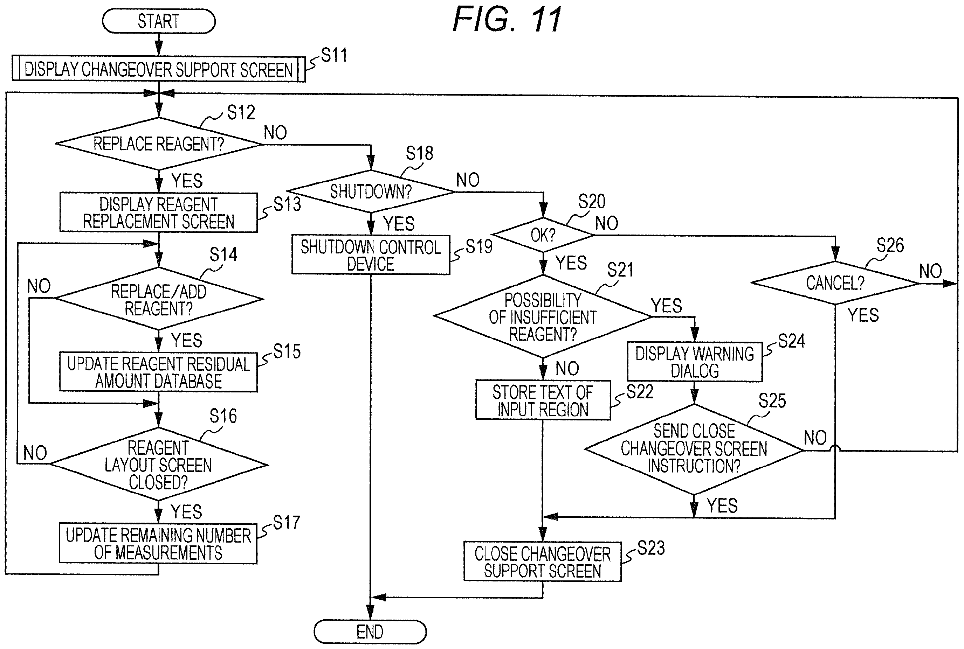

FIG. 11 is a flow chart showing the process performed by the control device 4 during the changeover. This process starts when the user presses the changeover button D15 of the menu screen D1.

The CPU 401 of the control device 4 performs the display process of the changeover support screen D7 when the changeover button D15 is pressed. The display process of the changeover support screen D7 is described below with reference to FIG. 12B. The CPU 401 performs the following process in accordance with the pressing of the reagent replacement button D713, shutdown button D722, OK button D75, or cancel button D76 by the user.

When the reagent replacement button D713 is pressed (S12: YES), the CPU 401 displays the reagent layout screen D2 on the display section 41. The user replenishes the reagent through the reagent layout screen D2 as described above. When the reagent replace/add button D26 is pressed on the reagent layout screen D2 (S14: YES), the CPU 401 updates the reagent residual amount database (S15). Specifically, the barcode of all reagent containers 103 on the reagent tables 11 and 12 are read by the barcode reader 14, and the pipette 18a is lowered and the residual amount of reagent is obtained for the reagent containers 103 which are replaced or added to, that is, reagent not recorded in the reagent residual amount database of FIG. 8A. In this way the reagent residual amount database is updated.

When the reagent layout screen D2 is closed (S16: YES), the CPU 401 updates the remaining measurement number items in the list D711 based on the updated reagent residual amount database, and updates the highlighted display of the list D711 and the display of the notification region D712 (S17). The process then returns to S12.

When the shutdown button D722 is pressed (S18: YES), the CPU 401 sets a flag to remind the CPU 401 to automatically display the maintenance screens D3 and D4 when the next user log on, and shuts down the control device 4 (S19). The user then replaces the lamp unit 20 or piercer 17a as described above.

When the OK button D75 is pressed (S20:YES), the CPU 401 determines whether there is a measurement item with a possibility of reagent insufficiency on the list D711 (S21). When a measurement item with a possibility of reagent insufficiency is not present (S21: NO), the CPU 401 stores the text data entered in the input region D74 on the hard disk 404 (S22), and a flag is set to remind the CPU 401 to automatically display a screen containing the stored text on the display section 41 when the next user logs on, whereupon the changeover support screen D7 closes (S23).

When a measurement item with a possibility of reagent insufficiency is present on the list D711 (S21: YES), the CPU 401 shows a warning dialog D8 on the display section 41 as shown in FIG. 12A (S24). The changeover support screen D7 is closed (S23) when the user presses the OK button D81 in the warning dialog D8 (S25: YES), or the process returns to S12 when the user presses the cancel button D82 in the warning dialog D8 (S25: NO).

When the cancel button D76 is pressed (S26: YES), the changeover support screen D7 is closed (S23).

FIG. 12B is a flow chart showing the display process of the changeover support screen D7 of S11 in FIG. 11.

The CPU 401 of the control device 4 obtains the residual amount of each reagent from the reagent residual amount database, and shows the remaining number of measurements corresponding to each measurement item in the list D711 based on the obtained residual amounts and the reagent usage amount settings (S101). The CPU 401 obtains the number of measurements for each measurement item in the night time period (18:00 hours to 09:00 hours next day) on the same day of the week one week prior, two weeks prior, and three weeks prior based on the measurement history database and displays the data on the list D711 (S102).

The CPU 401 then sets the maximum value of the past measurement number obtained in S102 as a threshold value Sh1 for each measurement item, and sets the value obtained by adding a predetermined value (for example, 2) to the threshold value Sh1 as the threshold value Sh2 (S103).

The CPU 401 then highlights the display of the remaining number of measurements and the measurement item based on the content shown in the list D711 (S104). Specifically, the measurement item and remaining number of measurements are highlighted in a dark color when the remaining number of measurements (in this case 5) is less than the threshold value Sh1 (in this case 8) as indicated by measurement item "ATIII" in FIG. 10. The measurement item and remaining number of measurements are highlighted in a light color when the remaining number of measurements (in this case 10) is greater than the threshold value Sh1 (in this case 9) but less than the threshold value Sh2 (in this case 11 since N=2) as indicated by measurement item "FDP".

The CPU 401 then shows, in the notification region D712, a message indicating a high possibility of reagent insufficiency (in this case, the reagent used in measurement item "ATIII"), and a message indicating a possibility of reagent insufficiency (in this case, reagent used in measurement item "FDP") of the measurement items highlighted in the list D711 (S105).

The CPU 401 then displays the information related to the lamp unit 20 and the piercer 17a stored on the hard disk 404 in the list D721 (S106). The CPU 401 also displays, on the list D73, the errors generated during logon by the current user, and the number of occurrences of the same generated error among errors generated during logon by the current user based on the error history database (S107).

The above is a flow of a series of operations when changing over operation of the day time user to the night time user. Although the example shows "night time" selected in the screen D6 of FIG. 9, the basic flow does not change even when different content is displayed in the list D711 of the changeover support screen D7 when "holiday" is selected. When "holiday" is selected, the measurement history is extracted from 00:00 hours Saturday to 24:00 hours Sunday of the prior week, two weeks prior, and three weeks prior from the measurement history database, the number of measurements of each measurement item are aggregated, and displayed on the list D711.

After reagent replenishment is completed and the daytime user has logged off the control device 4, the night time user who takes over operation from the daytime user can use the sample analyzer 1 by logging on the control device 4 in the same way as the daytime user. When examination is performed by the night time user, the history of these examinations is stored successively in the measurement history database together with the measurement time and date. When performing the next changeover, the stored measurement history becomes the most recent measurement history displayed on the list D711 of the changeover support screen D7.

When the work shift is completed or there is no need to wait for an urgent sample, the night time user logs off the control device 4 or shuts down the control device 4 and turns off the measurement section 2.

According to the present embodiment, when the current user presses the changeover button D15 to change over the operation to another user, the residual amount of each reagent is obtained from the reagent residual amount database, and the total number of remaining measurements of each measurement item is displayed on the list D711 based on the obtained residual amounts and the reagent usage amount settings. The current user therefore can easily and smoothly comprehend the number of remaining measurements of each measurement item. Since the reagent required for a measurement item is determined as shown in the reagent usage amount setting of FIG. 8B, the current user can be aware of the possibility of insufficient reagent according to the number of remaining measurements, and can determine whether sufficient reagent remains for the duty time of the post-changeover user. Since the current user can suitably replenish reagent before the changeover to another user, the need for replenishing reagent can be avoided after the changeover.

According to the present embodiment, the current user can smoothly determine whether to add or replace reagent, and whether to replace consumables through the changeover support screen D7. Therefore, the current user presses the reagent information button D13 on the menu screen D1 and the reagent layout screen D2 is displayed, the user determines whether to replace or add to each reagent and presses the maintenance button D14 of the menu screen D1 to display the maintenance screens D3 and D4, then determines whether to replace the consumables without performing any complex operations. The current user thus can smoothly determine whether to add or replace reagent, and whether to replace consumables.

According to the present embodiment, the number of measurements of each measurement item is obtained for past time periods identical to the time period of the changeover, and the obtained number of past measurements of each measurement item is shown in the list D711. In this way the user can empirically determine whether the number of remaining measurements of each measurement item is sufficient within the operating time of the post-changeover user by comparing the number of remaining measurements of each measurement item and the number of past measurements of each measurement item. The user can easily determine whether to add or replace reagent since the user is aware of the possibility of insufficient reagent based on whether there is sufficient number of remaining measurements.

According to the present embodiment, the number of past measurements of each measurement item is respectively associated with the residual number of measurements of each measurement item and displayed in the list D711. In this way the user can easily compare the number of past measurements and the number of remaining measurements since the number of past measurements and the number of remaining measurements are associated and displayed.

According to the present embodiment, three time periods (one week prior, two weeks prior, and three weeks prior) similar to the time period of the changeover are set as the past time periods identical to the time period of the changeover. In this way the user can accurately know whether the number of remaining measurements is sufficient since the user comprehends the changes of the past number of measurements.

According to the present embodiment, the measurement item and the number of remaining measurements are highlighted in a dark color when the number of remaining measurements is less than the threshold value Sh1 indicating the maximum value of the number of past measurements, and the measurement item and number of remaining measurements are highlighted in a light color when the number of remaining measurements is equal to or greater than the threshold value Sh1 but less than the threshold value Sh2 (threshold value Sh1+N). A display identical to the highlighted display is shown in the notification region D712. In this way the user can easily be aware of the measurement item with insufficient number of remaining measurements, and can smoothly add or replace reagent since the reagent with a possibility of insufficiency is easily determined.

According to the present embodiment, the user can set the time period of the changeover for the operation of the sample analyzer 1 through the screen D6, and the number of measurements in the same time period of the past are display on the list D711 according to the specified time period. In this way the user can estimate the amount of usage of reagent after the changeover more closely since the user comprehends the number of measurements in the same time periods of the past.

According to the present embodiment, the screen D6 includes a check box D61 corresponding to night time, and a check box D62 corresponding to holiday. When both night time and holiday operation are applicable, the changeover to night time operation or the changeover to holiday operation of the sample analyzer 1 can be simply specified.

According to the present embodiment, text entered in the input region D74 when the post-changeover user logs on is shown on the display section 41. Cautionary items related to apparatus operation, and specifically preparations, can therefore be communicated to the post-changeover user who is unfamiliar with the operation.

According to the present embodiment, errors generated during the logon of the current user are obtained from the error history database, and the obtained errors are shown on the list D73. In this way the user can refer to the list D73 and know, for example, that there is a possibility similar error generation after the changeover. The pre-changeover user can take steps to prevent the failure during post-changeover operation and can leave a communication for the post-changeover user in case a failure occurs after the changeover. The pre-changeover user can smoothly respond when there have been inquiries of similar errors from the post-changeover user.

Since the changeover button D15 is provided on the menu screen D1 in the present embodiment, the user can confirm the number of remaining measurements of each measurement item with a predetermined timing before the changeover, and, hence, confirm reagent that is possibly insufficient and consumables needing replacement.

According to the present embodiment, the user presses the reagent replacement button D713 to display the reagent layout screen D2 when the user determines reagent replacement or addition is required by glancing at the list D711. In this way the user smoothly handles the replacement or addition of reagent through the reagent layout screen D2.

According to the present embodiment, the list D721 shows the service life period and usage time of the lamp unit 20 and the number of possible uses and number of times used of the piercer 17a. In this way the current user knows before the changeover that the usage time of the lamp unit 20 will soon attain the end of the service life period, or the number of times used of the piercer 17a has reached the number of possible uses. Therefore, replacing the part beforehand reduces the risk of a breakdown during night time or holiday operation to the lowest level, and the workaround of preparing parts ahead of time is useful for the post-changeover user to easily replace a part.

### Modifications

When the changeover button D15 is pressed in the above embodiment, the screen D6 is displayed as shown in FIG. 9, and the user selects the time period to change over operation. In another embodiment of the present invention, the control device 4 may automatically determine the time period of the changeover based on time when the changeover button D15 is pressed.

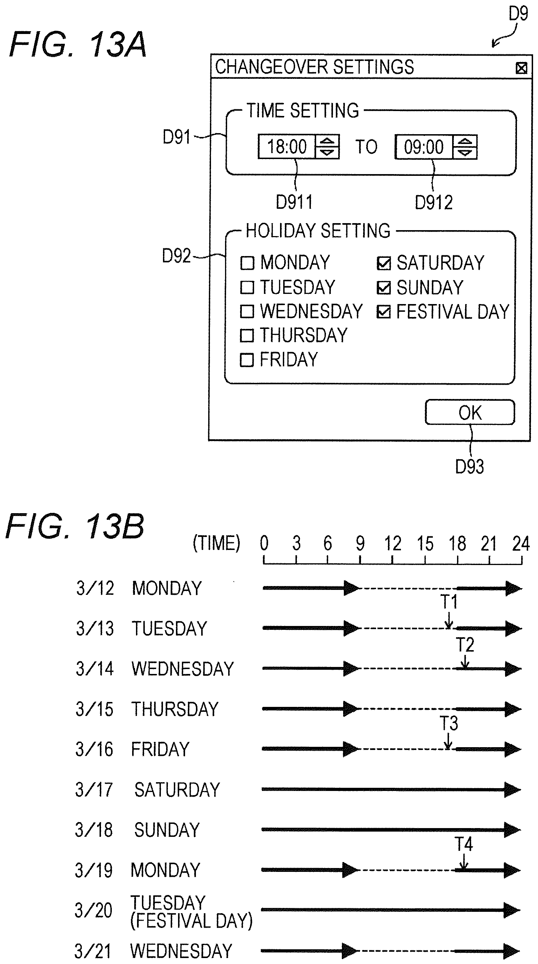

FIG. 13A shows the changeover setting screen D9 being displayed on the display section 41 of the control device 4.

The changeover setting screen D9 is displayed via a display instruction in the instruction screen shown by pressing the setting button D18 of the menu screen D1. The changeover setting screen D9 includes a region D91 for the time setting, region D92 for the holiday setting, and OK button D93.

The start and end times of the time period (night time period of days other than holidays) included in the night time period and the like are respectively entered in the input boxes D911 and D912. The day (day set to holiday) included in the night time period is set in the check box in region D92. When the setting of regions D91 and D92 are completed and the OK button D93 is pressed, the content set in regions D91 and D92 are stored on hard disk 404.

FIG. 13B shows an example of time periods of night time and holiday of a particular period (March 12 to March 21) when the settings are performed as shown in FIG. 13A.

Since Monday through Friday is not set as holiday in region D92, Monday through Friday is recognized as weekday. Also, the period from 18:00 hours to 09:00 hours is set as the night time period according to the region D91. Accordingly, night time periods of weekdays, i.e., time periods from 18:00 hours to 09:00 hours of each of March 12 to March 16 and March 19 to March 21 are determined as the night time periods. Also, 24 hours of March 17, March 18, and March 20 are determined as the night time period since Saturday, Sunday, and festival day are set as holidays in region D92.

With regard to setting time period as described above, when the user performing the changeover presses the changeover button D15, on the basis of the current time when the button D15 is pressed, the upcoming night time period or the night time period including the present time (hereinafter referred to as "changeover time period") is determined.

For example, when the timing of pressing the changeover button D15 is time T1, the changeover time period is determined as 18:00 hours on March 13 to 09:00 hours on March 14. When the timing of pressing the changeover button D15 is time T2, the changeover time period is determined as 18:00 hours on March 14 to 09:00 hours on March 15. When the timing of pressing the changeover button D15 is time T3, the changeover time period is determined as 18:00 hours on March 16 to 09:00 hours on March 19. When the timing of pressing the changeover button D15 is time T4, the changeover time period is determined as 18:00 hours on March 19 to 09:00 hours on March 21.

Since the time period of the changeover is automatically determined in this embodiment, the user selection error in screen D6 of FIG. 9 is eliminated, and reagent preparation can be more reliably implemented. The user also can set the night time period and the like through the screen D6. In this way the time period for obtaining and displaying the past number of measurements can be set to a time period corresponding to various changeover timings of each facility.

In the above embodiment, although the pre-changeover user specified the time period for use of the apparatus by selecting one time period from among several predefined time periods in screen D6 of FIG. 9, the present invention is not limited to this selection inasmuch as the user also may specify a time period by numerical value.

Although this embodiment by way of an example of selection between "night" and "holiday" as the time period of the next changeover of apparatus operation on the screen D6 of FIG. 9, "day" also may be an additional selection. In the example of FIG. 9, Since night time is defined as 18:00 hours to 09:00 hours the next day, the day time period may be defined, for example, as 09:00 hours to 18:00 hours so as to combine day and night definitions as 24 hours without mutual overlap.

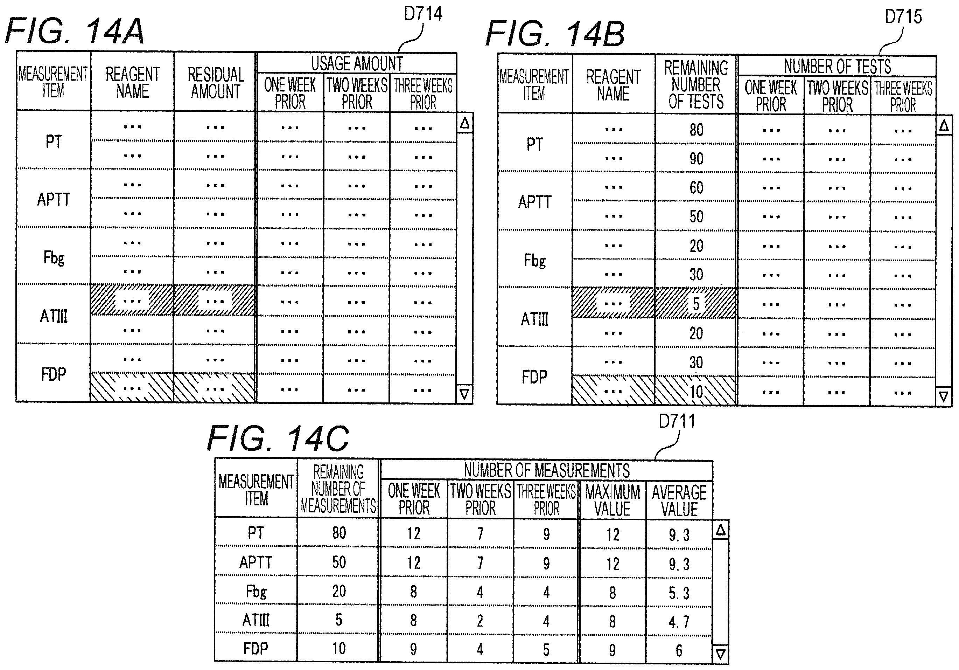

Although the number of remaining measurements and the number of past measurements are displayed for each measurement item in the list D711 of the changeover support screen D7 in the above embodiment, this information also may be displayed in the list D714 shown in FIG. 14A or the list D715 shown in FIG. 14B rather than in list D711.

The list D714 shows the reagent name, residual amount of reagent, and past usage amount for each measurement item. Here, residual amount does not refer to the number of remaining measurements that can be performed by the reagent, rather it refers to information regarding the volume of the remaining reagent (for example, how many milliliters of reagent remain). The past usage amount is information relating to the amount of reagent used in the past for that measurement item, that is, the number of milliliters used. The maximum values of the residual amount and past usage amount are compared, and the reagent name and residual amount of reagent that have been determined to have a high possibility of being insufficient are highlighted in dark color. Values obtained by adding a predetermined value M to the maximum values of the residual amount and the past usage amount are compared, the reagent name and residual amount of reagents that have been determined to have a possibility of being insufficient are highlighted in light color.

The reagent name, number of remaining tests of the reagent, and past number of tests performed are displayed for each measurement item in the list D715. The number of remaining tests is the number of times a measurement item can be measured using the reagent, and the past number of tests is the number of past measurements of a measurement item that have been measured using the reagent. The maximum values of the residual number of tests and past number of tests are compared, and the reagent name and residual number of tests of reagent that have been determined to have a high possibility of being insufficient are highlighted in dark color. Values obtained by adding a predetermined value M to the maximum values of the residual number of tests and the past number of tests are compared, and the reagent name and residual number of tests of reagents that have been determined to have a possibility of being insufficient are highlighted in light color.

When the lists D714 and D715 are displayed, the name of reagents having a possibility of being insufficient are displayed in the notification region D712. In this case the display process of the changeover support screen D7 is substantially similar to the display process of the changeover support screen D7 of the previous embodiment (refer to FIG. 12B). The user determines whether to add or replace reagent similar to the above embodiment by glancing at the displayed lists D714 and D715.

Although described by way of the above embodiments, the present invention is not limited to these embodiments and may be variously modified.

For example, although the sample analyzer 1 is a blood coagulation analyzer in the above embodiment, the present invention is not limited to this application inasmuch as the sample analyzer 1 may be another type of apparatus for analyzing clinical sample. For example, the sample analyzer 1 may be an immunoanalyzer or biochemical analyzer for measuring blood serum, a blood cell counter for counting blood cells in a blood sample, urine analyzer for analyzing urine samples, or analyzer for analyzing bone marrow fluid.

The maximum value and average value of the number of past measurements also may be displayed as shown in FIG. 14C in the list D711 of the above embodiment. In this case if the number of residual measurements exceeds the maximum value of the number of past measurements, the user can determine that a need to replace or replenish reagent will not occur after the changeover. If the number of residual uses exceeds the average value of the number of past measurements, the user can determine that a need to replace or add reagent is empirically unlikely to occur after the changeover.

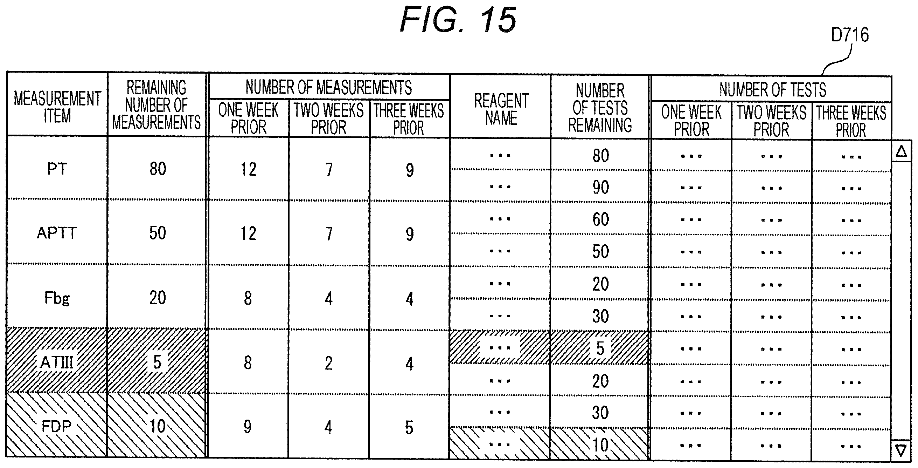

The list D711 of the above embodiment and the lists D714 and D715 of the modification also may be displayed together on the changeover support screen D7. In this case the display content of the list D714 may be combine with the display content of the list D711. As shown in FIG. 15C, the display content of list D715 may be combined with the display content of the list D711 as a list D716. In this way when the information related to the residual amount of reagent is combine and displayed with the remaining number of measurement items, the information related to the residual amount of reagent associated with the measurement item can be confirmed.

Although the changeover support screen D7 is displayed when the changeover button D15 is pressed in the above embodiment, the present invention is not limited to this arrangement inasmuch as the changeover support screen D7 is displayed when the user ends the operation of the sample analyzer 1, that is, when the logoff button D16 or the shutdown button D17 is pressed. In this way the residual number of measurements, service life period and usage time of the lamp unit 20, and number of possible uses and number of uses of the piercer 17a can be displayed when the user ends the operation of the sample analyzer 1. Since the user can reliably comprehend the need to replace components and replenish the reagent and consumables, a condition in which reagent replenishment and component replacement is required can be avoided more reliably.

In the above embodiment, the amount of consumed reagent during a predetermined time period (18:00 to 09:00 for night time)is displayed as history information related to the consumption amount of reagent or consumable consumed in a predetermined past time period.

The "predetermined time period" in the present invention includes not only periods repeated at fixed intervals (for example, 18:00 to 09:00 daily), but also indefinite periods and periods uniquely defined by certain conditions. For example, the "predetermined time period" of the present invention belongs to various categories such as a period from a particular operation of the device to the next operation, a period in which a particular mode of the device is set, a period in which an operator having a particular authority is logged on the device. An example in which a period defined by particular conditions is used as the predetermined time period is described below.

For example, at least two authority levels of "general operator" and "night operator" may be set as the user authorities for operating the sample analyzer 1. A "general operator" has the authority allocated to a user who operates the apparatus during the daytime period, and can use almost all the functions of the apparatus. A "night operator" has the authority allocated to a user who operates the apparatus during the night time period, and can only operate the apparatus within a range of operations that includes only sample measurements and replenishing and replacing reagent and the like. One of the two authority levels is assigned to each user and the account assigned to each user corresponds to one of the two authority levels.

Since some users assigned with the "night operator" authority are unfamiliar with the sample analyzer 1, the functions available under the "night operator" authority is restricted in a reasonable scope in order to avoid an erroneous operation of the apparatus due to undesired operation and a breakdown.

When user logs on the control device 4, the user authority is determined as "general operator" or "night operator" on the basis of his account. When a sample examination is performed, the history of the examination is stored associated with the authority level of the log on user. When displaying history information related to the consumption amount of reagent or consumable, the examination history associated with the authority level of "night operator" is extracted from the examination history, and the consumption amount of the reagent consumed during the night time period is calculated.

In another mode the sample apparatus 1 does not perform user management. User management includes establishing an account for each user, and the apparatus recognizing who has logged on by entering an account during logon.

In the mode of no user management, the sample analyzer 1 is operated in a different mode according to the work day or time period in which the apparatus is operating. Specifically, when the user starts the apparatus, she must select either "day mode" or "night mode." Day mode is a mode where almost all functions of the apparatus are available. Night mode is a mode where a range of available functions is limited in comparison with the day mode to specific functions including measuring samples and replenishing and replacing reagent. For example, calibration of the sample analyzer 1 is authorized in the day mode, but it is not authorized in night mode.

When the sample analyzer 1 is started, the user selects to start the apparatus in either the day mode or the night mode. When an examination is performed after starting the apparatus, the examination history is stored associated with the mode type under which the examination was performed. When displaying history information related to the consumption amount of reagent or consumable, the examination history performed under the "night mode" is extracted from the examination history, and the consumption amount of the reagent consumed during the night time period is calculated.

Although the notification region D712 displays a notification message indicating a high possibility of insufficient reagent in the above embodiment, the present invention is not limited to this arrangement inasmuch as a message urging the user to replace and add reagent also may be displayed.

In the above embodiment, the reagent residual amount database, reagent usage amount setting, error history database, measurement history database, service life period and usage time of the lamp unit 20, and number of possible uses and number of times used of the piercer 17a are stored on the hard disk 404 of the control device 4. However, this information also may be stored in the memory 202 of the measurement section 2, and may be stored on an external host computer connected to the sample analyzer 1 with communication enabled.

Although the reagent residual amount database is stored on the hard disk 404 of the control device 4 in the above embodiment, other modes are also possible. For example, an RFID which is a readable/writable data carrier may be provided on the outside of the reagent container 103, and the reagent residual amount may be stored on the RFID. In this mode, the reagent residual amount stored on the RFID is updated every time reagent is consumed, and the reagent residual amount is read from each reagent container 103 and a screen is generated when the changeover support screen D7 of FIG. 10 is displayed.

Although the reagent residual amount is stored in volume order in the reagent residual amount database, and the reagent residual amount database is updated by subtracting the aspirated amount from the recorded amount in the above embodiment, the present invention is not limited to this method. The reagent residual amount database also may be configured to manage the residual amount by decrementing 1 from the number of remaining tests predetermined for each reagent container 103 each time reagent is aspirated.

Although the past time period is set at one week prior, two weeks prior, and three weeks prior for the changeover time period in the above embodiment, the present invention is not limited to this arrangement. For example, when the changeover time period is a weekday, the past time period may be the most recent three time periods from among the changeover time periods of past weekdays.

In the above embodiment, the maximum value of the number of measurements in the past time period was designated threshold Sh1, the value obtained by adding a predetermined value to the threshold Sh1 was designated threshold Sh2, and the highlighted display of the list D711 and the display of the notification region D712 were performed based on the thresholds Sh1 and Sh2. However, when considering the number of remaining measurements which are desired to remain normally, a fixed threshold Sh3 also may be set relative to each measurement item.

Although the reagent replacement button D713 is pressed to display the reagent layout screen D2 when reagent replacement and addition are determined to be necessary in the above embodiment, alternatively, the reagent layout screen D2 may be displayed when the name of the reagent requiring replacement or addition is pressed in the list D14 through D16 when the lists D14 through D16 are being displayed. In this case, the reagent container 103 requiring replacement or addition on the reagent tables 11 and 12 are positioned directly below the cover 2a.

Although the number of residual uses of reagent is displayed in the list D711 of FIG. 14B, the present invention is not limited to this arrangement inasmuch as the number of remaining uses and the residual value of other consumables also may be displayed. Consumables may be the cuvettes stored in the cuvette supplier 15, or washing liquid for washing the piercer 17a and pipette 18a. In the case of a sample analyzer which aspirates samples by with a disposable pipette tip, the pipette tip is another example of a consumable item. In the case of an apparatus using a slide on which a sample is smeared, the slide is yet another example of a consumable. In this case, a button similar to the reagent replacement button D713 is provided in the changeover support screen D7, so that replenishment of other consumables can be performed by pressing this button.

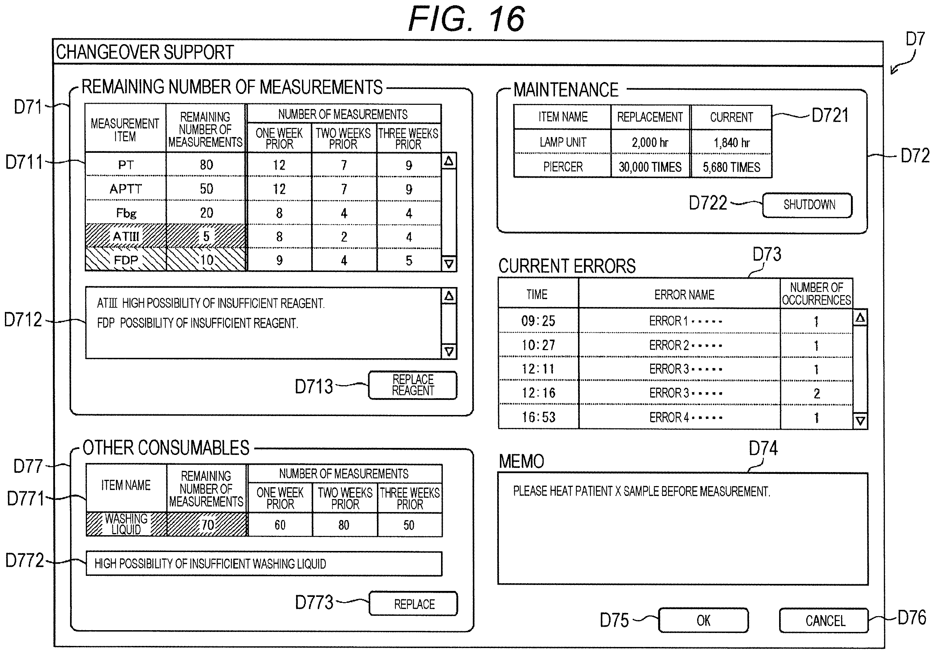

FIG. 16 shows the changeover support screen D7 when it includes the region D77 for showing information related to washing liquid. The region D77 includes a list D771, notification region D772, and replacement button D773. Note that the part outside the region D77 is substantially similar to the changeover support screen shown in FIG. 10.

In the list D771, the number of residual measurements is the number of measurement possible using the washing liquid currently available, and the number of past measurements is the number of measurements performed using the washing liquid during the night time periods on the same day one week prior, two weeks prior, and three weeks prior to the present day. Note that a float sensor for detecting the liquid surface position of the washing liquid in the container is provided in a tube connected to the container which holds the washing liquid, and the amount of the remaining washing liquid is obtained based on the detection signal of the float sensor. The number of remaining measurements in the list D771 is a value obtained by dividing the obtained residual amount of washing liquid by a predetermined value. The predetermined value is determined based on the amount of washing liquid used in one measurement, which is different for each measurement item.

Since the remaining number of measurements is less than the maximum value (80) of the number of past measurements in the list D771, the number of measurement and the item name are highlighted in dark color, and a notification message indicating the high possibility of insufficient washing liquid is displayed in the notification region D772. When the user determines there is a possibility of insufficient washing liquid by referring to the list D771 and the notification region D772, the user presses the replacement button D773 and a screen for performing a replacement or extension of washing liquid is displayed. When the washing liquid replacement or extension is completed and the screen for replacement or extension is closed, the display content of the region D77 is updated.

These other embodiments of the present invention are not limited to the above described embodiments and may be variously modified insofar as such modification are within the scope of the claims.

## Claims

1. A sample analyzer (1) comprising:
a storing means for storing a reagent or consumable used for measuring a sample;
a measuring means (2) for measuring a sample using the stored reagent or consumable;
a control device (4) for analyzing measurement data obtained by the measurement means (2) and issuing instructions for the measurement means (2); and
a displaying means (41) for displaying a screen, wherein
when a current user logs onto the control device (4), the displaying means (41) is configured to display a menu screen (D1) including a logoff button (D16), **characterized in that**, when the current user presses the logoff button (D16), the displaying means (41) is configured to display a changeover support screen (D7) for helping a changeover of operation of the sample analyzer to another user, the support screen showing a residual amount information (D71) related to an amount of remaining reagent or consumable stored in the measurement means.

2. The sample analyzer (1) of claim 1, **characterized in that**
the changeover support screen (D7) further includes a performance information relating to a consumption performance of reagent or consumable consumed by the measuring means in a predetermined time period in a past.

3. The sample analyzer (1) of claim 2, further comprising
a means for determining whether the residual amount information is in a predetermined relationship with the performance information, and
**characterized in that** the changeover support screen (D7) includes an information display region for showing a residual amount information and a performance information, and an alert display region for showing an alert, and
the displaying means (41) shows an alert related to an insufficiency of the reagent or consumable when the residual amount information is not in the predetermined relationship.

4. The sample analyzer (1) of claim 2 or 3, **characterized in that**
the storing means stores a plurality of reagents or a plurality of consumables, and
the changeover support screen (D7) includes a performance information and a residual amount information for each of the reagents or consumables stored by the storing means.

5. The sample analyzer (1) of any one of claims 2 to 4, further comprising
an obtaining means for obtaining the consumption performance of the reagent or consumable that have been consumed by the measuring means during a plurality of time periods, and
a generating means for generating the changeover support screen using an average value of the consumption performances or a maximum value among the consumption performances obtained by the obtaining means.

6. The sample analyzer (1) of any one of claims 2 to 5, **characterized in that**
the displaying means (41) highlights the residual amount information when the residual amount of the reagent or consumable does not meet a predetermined threshold value or a threshold value determined based on the consumption performance.

7. The sample analyzer (1) of any one of claims 2 to 6, **characterized in that**
the performance information is an amount of reagent or consumable which is consumed by the measuring means or a number of measurements performed using the reagent or consumable performed by the measuring means in the predetermined time period.

8. The sample analyzer (1) of any one of claims 2 to 7, **characterized in that**
the displaying means (41) is also enabled to display a specification screen for specifying the time period where the sample analyzer is operated after the changeover; and
the displaying means (41) displays, as the performance information, the consumption performance of the reagent or consumable consumed by the measuring means during the time period specified on the specification screen.

9. The sample analyzer (1) of claim 8, **characterized in that**
the specification screen includes as selection options at least a first time period defined as night time and a second time period defined as holiday;
the displaying means (41) displays the consumption performance of the reagent or consumable consumed by the measuring means during the first time period of a past day as the performance information, when the first time period is selected in the specification screen, and
the displaying means (41) displays the consumption performance of the reagent or consumable consumed by the measuring means during the second time period in a past as the performance information, when the second time period is selected in the specification screen.

10. The sample analyzer (1) of any one of claims 1 to 9, **characterized in that**
the changeover support screen (D7) includes a message input region for inputting a message for the user operating the sample analyzer after changeover.

11. The sample analyzer (1) of any one of claims 1 to 10, **characterized in that**
the changeover support screen (D7) includes an error display region for showing information related to failures occurring in the sample analyzer on the day.

12. The sample analyzer (1) of any one of claims 1 to 11, **characterized in that**
the measuring means includes a part required to be periodically replaced with a new one,
the changeover support screen (D7) includes a part information region for displaying a durable period of the part or a number of uses of the part, and an elapsed time from an installation of the part or a number of uses of the part.

13. The sample analyzer (1) of any one of claims 1 to 12, **characterized in that**
the displaying means (41) displays the changeover support screen when a button for completing a work is operated.

14. The sample analyzer (1) of any one of claims 1 to 13, **characterized in that**
the residual amount information is a number of measurements which can be performed by the measuring means using the remaining reagent or consumable.

15. The sample analyzer (1) of any one of claims 1 to 14, **characterized in that**
the changeover support screen (D7) includes a replenishment button, and
the displaying means (41) shows a replenishment screen on which information of a reagent or consumable to be replenished is input, when the replenishment button of the changeover support screen is operated.

## Patentansprüche

1. Probenanalysegerät (1), umfassend:
ein Speichermittel zum Speichern eines Reagenzmittels oder Verbrauchsmittels, das zum Messen einer Probe verwendet wird;
ein Messmittel (2) zum Messen einer Probe unter Verwendung des gespeicherten Reagenzmittels oder Verbrauchsmittels;
eine Steuervorrichtung (4) zum Analysieren von Messdaten, die von dem Messmittel (2) erhalten werden, und Ausstellen von Befehlen für das Messmittel (2); und
ein Anzeigemittel (41) zum Anzeigen eines Bildschirms, wobei
wenn ein aktueller Benutzer sich auf der Steuervorrichtung (4) anmeldet, das Anzeigemittel (41) dazu ausgelegt ist, einen Menübildschirm (D1) anzuzeigen, der eine Abmeldeschaltfläche (D16) beinhaltet,
**dadurch gekennzeichnet, dass**, wenn der aktuelle Benutzer die Abmeldeschaltfläche (D16) drückt, das Anzeigemittel (41) dazu ausgelegt ist, einen Übergabeunterstützungsbildschirm (D7) anzuzeigen, um bei einer Betriebsübergabe des Probenanalysegeräts an einen anderen Benutzer zu helfen, wobei der Unterstützungsbildschirm eine Restmengeninformation (D71) bezüglich einer Menge verbleibenden Reagenzmittels oder Verbrauchsmittels, das in dem Messmittel gespeichert ist, zeigt.

2. Probenanalysegerät (1) nach Anspruch 1, **dadurch gekennzeichnet, dass**
der Übergabeunterstützungsbildschirm (D7) weiter eine Leistungsinformation bezüglich einer Verbrauchsleistung von Reagenzmittel oder Verbrauchsmittel beinhaltet, das von dem Messmittel in einer vorgegebenen Zeitdauer in einer Vergangenheit verbraucht wurde.

3. Probenanalysegerät (1) nach Anspruch 2, weiter umfassend
ein Mittel zum Bestimmen, ob die Restmengeninformation in einer vorgegebenen Beziehung zu der Leistungsinformation steht, und
**dadurch gekennzeichnet, dass** der Übergabeunterstützungsbildschirm (D7) einen Informationsanzeigebereich, um eine Restmengeninformation und eine Leistungsinformation anzuzeigen, und einen Warnanzeigebereich beinhaltet, um eine Warnung anzuzeigen, und
das Anzeigemittel (41) eine Warnung bezüglich eines Mangels an dem Reagenzmittel oder Verbrauchsmittel anzeigt, wenn die Restmengeninformation nicht in der vorgegebenen Beziehung steht.

4. Probenanalysegerät (1) nach Anspruch 2 oder 3, **dadurch gekennzeichnet, dass**
das Speichermittel eine Vielzahl von Reagenzmitteln oder eine Vielzahl von Verbrauchsmitteln speichert, und
der Übergabeunterstützungsbildschirm (D7) eine Leistungsinformation und eine Restmengeninformation für jedes der Reagenzmittel oder Verbrauchsmittel beinhaltet, die von dem Speichermittel gespeichert werden.

5. Probenanalysegerät (1) nach einem der Ansprüche 2 bis 4, weiter umfassend
ein Bezugsmittel zum Erhalten der Verbrauchsleistung des Reagenzmittels oder Verbrauchsmittels, das von dem Messmittel während einer Vielzahl von Zeitdauern verbraucht wurde, und
ein Erzeugungsmittel zum Erzeugen des Übergabeunterstützungsbildschirms unter Verwendung eines Durchschnittswerts der Verbrauchsleistungen oder eines Höchstwerts unter den Verbrauchsleistungen, die von dem Bezugsmittel erhalten wurden.

6. Probenanalysegerät (1) nach einem der Ansprüche 2 bis 5, **dadurch gekennzeichnet, dass**
das Anzeigemittel (41) die Restmengeninformation hervorhebt, wenn die Restmenge des Reagenzmittels oder Verbrauchsmittels einen vorgegebenen Schwellenwert oder einen Schwellenwert, der basierend auf der Verbrauchsleistung bestimmt wird, nicht erreicht.

7. Probenanalysegerät (1) nach einem der Ansprüche 2 bis 6, **dadurch gekennzeichnet, dass**
die Leistungsinformation eine Menge an Reagenzmittel oder Verbrauchsmittel, die von dem Messmittel verbraucht wird, oder eine Anzahl von Messungen ist, die unter Verwendung des Reagenzmittels oder Verbrauchsmittels von dem Messmittel in der vorgegebenen Zeitdauer durchgeführt wird.

8. Probenanalysegerät (1) nach einem der Ansprüche 2 bis 7, **dadurch gekennzeichnet, dass**
das Anzeigemittel (41) auch dazu fähig ist, einen Spezifikationsbildschirm anzuzeigen, um die Zeitdauer zu spezifizieren, in der das Probenanalysegerät nach der Übergabe betrieben wird; und
das Anzeigemittel (41) als die Leistungsinformation die Verbrauchsleistung des Reagenzmittels oder Verbrauchsmittels anzeigt, das von dem Messmittel während der Zeitdauer verbraucht wird, die auf dem Spezifikationsbildschirm spezifiziert ist.

9. Probenanalysegerät (1) nach Anspruch 8, **dadurch gekennzeichnet, dass**
der Spezifikationsbildschirm als Auswahlmöglichkeiten zumindest eine erste Zeitdauer, die als Nachtzeit definiert ist, und eine zweite Zeitdauer, die als Feiertag definiert ist, beinhaltet;
das Anzeigemittel (41) die Verbrauchsleistung des Reagenzmittels oder Verbrauchsmittels, das von dem Messmittel während der ersten Zeitdauer eines vergangenen Tags verbraucht wurde, als die Leistungsinformation anzeigt, wenn die erste Zeitdauer in dem Spezifikationsbildschirm ausgewählt wird, und
das Anzeigemittel (41) die Verbrauchsleistung des Reagenzmittels oder Verbrauchsmittels, das von dem Messmittel während der zweiten Zeitdauer in einer Vergangenheit verbraucht wurde, als die Leistungsinformation anzeigt, wenn die zweite Zeitdauer in dem Spezifikationsbildschirm ausgewählt wird.

10. Probenanalysegerät (1) nach einem der Ansprüche 1 bis 9, **dadurch gekennzeichnet, dass**
der Übergabeunterstützungsbildschirm (D7) einen Nachrichteneingabebereich zum Eingeben einer Nachricht für den Benutzer, der das Probenanalysegerät nach der Übergabe betreibt, beinhaltet.

11. Probenanalysegerät (1) nach einem der Ansprüche 1 bis 10, **dadurch gekennzeichnet, dass**
der Übergabeunterstützungsbildschirm (D7) einen Fehleranzeigebereich zum Zeigen von Informationen bezüglich Störungen beinhaltet, die in dem Probenanalysegerät an dem Tag auftreten.

12. Probenanalysegerät (1) nach einem der Ansprüche 1 bis 11, **dadurch gekennzeichnet, dass**
das Messmittel ein Teil beinhaltet, das regelmäßig durch ein neues ersetzt werden muss,
der Übergabeunterstützungsbildschirm (D7) einen Teilinformationsbereich zum Anzeigen einer Haltbarkeitsdauer des Teils oder eine Anzahl von Verwendungen des Teils und einer vergangenen Zeit seit einer Installation des Teils oder einer Anzahl von Verwendungen des Teils beinhaltet.

13. Probenanalysegerät (1) nach einem der Ansprüche 1 bis 12, **dadurch gekennzeichnet, dass**
das Anzeigemittel (41) den Übergabeunterstützungsbildschirm anzeigt, wenn eine Schaltfläche zum Abschließen einer Arbeit betätigt wird.

14. Probenanalysegerät (1) nach einem der Ansprüche 1 bis 13, **dadurch gekennzeichnet, dass**
die Restmengeninformation eine Anzahl von Messungen ist, die von dem Messmittel unter Verwendung des verbleibenden Reagenzmittels oder Verbrauchsmittels durchgeführt werden können.

15. Probenanalysegerät (1) nach einem der Ansprüche 1 bis 14, **dadurch gekennzeichnet, dass**
der Übergabeunterstützungsbildschirm (D7) eine Nachfüllschaltfläche beinhaltet, und
das Anzeigemittel (41) einen Nachfüllbildschirm zeigt, auf dem Informationen über ein nachzufüllendes Reagenzmittel oder Verbrauchsmittel eingegeben werden, wenn die Nachfüllschaltfläche des Übergabeunterstützungsbildschirms betätigt wird.

## Revendications

1. Analyseur d'échantillon (1) comprenant :
un moyen de stockage pour stocker un réactif ou un consommable utilisé pour mesurer un échantillon ;
un moyen de mesure (2) pour mesurer un échantillon en utilisant le réactif ou le consommable stocké ;
un dispositif de commande (4) pour analyser des données de mesure obtenues par le moyen de mesure (2) et délivrer des instructions pour le moyen de mesure (2) ; et
un moyen d'affichage (41) pour afficher un écran, dans lequel
lorsqu'un utilisateur actuel se connecte au dispositif de commande (4), le moyen d'affichage (41) est configuré pour afficher un écran de menu (D1) incluant un bouton de déconnexion (D16),
**caractérisé en ce que**, lorsque l'utilisateur actuel appuie sur le bouton de déconnexion (D16), le moyen d'affichage (41) est configuré pour afficher un écran de support de changement (D7) pour aider à un changement de fonctionnement de l'analyseur d'échantillon vers un autre utilisateur, l'écran de support montrant une information de quantité résiduelle (D71) liée à une quantité de réactif ou de consommable restant stocké dans le moyen de mesure.

2. Analyseur d'échantillon (1) selon la revendication 1, **caractérisé en ce que**
l'écran de support de changement (D7) inclut en outre une information de performance relative à une performance de consommation de réactif ou de consommable consommé par le moyen de mesure dans une période de temps prédéterminée dans le passé.

3. Analyseur d'échantillon (1) selon la revendication 2, comprenant en outre
un moyen pour déterminer si l'information de quantité résiduelle est dans une relation prédéterminée avec l'information de performance, et
**caractérisé en ce que** l'écran de support de changement (D7) inclut une région d'affichage d'information pour montrer une information de quantité résiduelle et une information de performance, et une région d'affichage d'alerte pour montrer une alerte, et
le moyen d'affichage (41) montre une alerte liée à une insuffisance de réactif ou de consommable lorsque l'information de quantité résiduelle n'est pas dans la relation prédéterminée.

4. Analyseur d'échantillon (1) selon la revendication 2 ou 3, **caractérisé en ce que**
le moyen de stockage stocke une pluralité de réactifs ou une pluralité de consommables, et
l'écran de support de changement (D7) inclut une information de performance et une information de quantité résiduelle pour chacun des réactifs ou consommables stockés par le moyen de stockage.

5. Analyseur d'échantillon (1) selon l'une quelconque des revendications 2 à 4, comprenant en outre
un moyen d'obtention pour obtenir la performance de consommation du réactif ou du consommable qui a été consommé par le moyen de mesure pendant une pluralité de périodes de temps, et
un moyen de génération pour générer l'écran de support de changement à partir d'une valeur moyenne de la performance de consommation ou d'une valeur maximale parmi les performances de consommation obtenues par le moyen d'obtention.

6. Analyseur d'échantillon (1) selon l'une quelconque des revendications 2 à 5, **caractérisé en ce que**
le moyen d'affichage (41) met en évidence l'information de quantité résiduelle lorsque la quantité résiduelle du réactif ou du consommable ne respecte pas une valeur de seuil prédéterminée ou une valeur de seuil déterminée sur la base de la performance de consommation.

7. Analyseur d'échantillon (1) selon l'une quelconque des revendications 2 à 6, **caractérisé en ce que**
l'information de performance est une quantité de réactif ou de consommable qui est consommé par le moyen de mesure ou un nombre de mesures effectuées en utilisant le réactif ou le consommable effectuées par le moyen de mesure dans la période de temps prédéterminée.

8. Analyseur d'échantillon (1) selon l'une quelconque des revendications 2 à 7, **caractérisé en ce que**
le moyen d'affichage (41) est également activé pour afficher un écran de spécification pour spécifier la période de temps pendant laquelle l'analyseur d'échantillon est actionné après le changement ; et
le moyen d'affichage (41) affiche, en tant qu'information de performance, la performance de consommation du réactif ou du consommable consommé par le moyen de mesure pendant la période de temps spécifiée sur l'écran de spécification.

9. Analyseur d'échantillon (1) selon la revendication 8, **caractérisé en ce que**
l'écran de spécification inclut comme options de sélection au moins une première période définie comme étant la nuit et une seconde période définie comme étant des vacances ;
le moyen d'affichage (41) affiche la performance de consommation du réactif ou du consommable consommé par le moyen de mesure pendant la première période d'un jour passé en tant qu'information de performance, lorsque la première période de temps est sélectionnée dans l'écran de spécification, et
le moyen d'affichage (41) affiche la performance de consommation du réactif ou du consommable consommé par le moyen de mesure pendant la seconde période de temps dans un passé en tant qu'information de performance, lorsque la seconde période de temps est sélectionnée dans l'écran de spécification.

10. Analyseur d'échantillon (1) selon l'une quelconque des revendications 1 à 9, **caractérisé en ce que**
l'écran de support de changement (D7) inclut une région d'entrée de message pour entrer un message pour l'utilisateur faisant fonctionner l'analyseur d'échantillon après un changement.

11. Analyseur d'échantillon (1) selon l'une quelconque des revendications 1 à 10, **caractérisé en ce que**
l'écran de support de changement (D7) inclut une région d'affichage d'erreur pour montrer une information relative à des pannes se produisant dans l'analyseur d'échantillon le jour.

12. Analyseur d'échantillon (1) selon l'une quelconque des revendications 1 à 11, **caractérisé en ce que**
le moyen de mesure inclut une pièce devant être remplacée périodiquement par une neuve,
l'écran de support de changement (D7) inclut une région d'information de pièce pour afficher une période durable de la pièce ou un nombre d'utilisations de la pièce, et un temps écoulé depuis une installation de la pièce ou un nombre d'utilisations de la pièce.

13. Analyseur d'échantillon (1) selon l'une quelconque des revendications 1 à 12, **caractérisé en ce que**
le moyen d'affichage (41) affiche l'écran de support de changement lorsqu'un bouton pour terminer un travail est actionné.

14. Analyseur d'échantillon (1) selon l'une quelconque des revendications 1 à 13, **caractérisé en ce que**
l'information de quantité résiduelle est un nombre de mesures qui peuvent être effectuées par le moyen de mesure en utilisant le réactif ou le consommable restant.

15. Analyseur d'échantillon (1) selon l'une quelconque des revendications 1 à 14, **caractérisé en ce que**
l'écran de support de changement (D7) inclut un bouton de réapprovisionnement, et
le moyen d'affichage (41) montre un écran de réapprovisionnement sur lequel une information d'un réactif ou d'un consommable à réapprovisionner est entrée, lorsque le bouton de réapprovisionnement de l'écran de support de changement est actionné.
